# EUROPÄISCHE PATENTSCHRIFT

(11) **EP 3 609 617 B1**
(45) Veröffentlichungstag und Bekanntmachung des Hinweises auf die Patenterteilung: **09.06.2021**
(21) Anmeldenummer: 18716317.5
(22) Anmeldetag: 13.04.2018
(51) Int. Cl.: B01L 3/00

(54) **VORRICHTUNG ZUR PROZESSIERUNG EINER FLÜSSIGEN PROBE**
DEVICE FOR PROCESSING A LIQUID SAMPLE
DISPOSITIF DE TRAITEMENT D'UN ÉCHANTILLON LIQUIDE

(30) Priorität: 13.04.2017 LU 100171
(43) Veröffentlichungstag der Anmeldung: 19.02.2020
(73) Patentinhaber: cytena Bioprocess Solutions co., Ltd., Taipei City 110 (TW)
(72) Erfinder: SCHÖNDUBE, Jonas, 79108 Freiburg (DE); ZIMMERMANN, Stefan, 79283 Bollschweil (DE); GROSS, Andre, 79104 Freiburg (DE); CHENGHAN, Tsai, 79098 Freiburg (DE); KOLTAY, Peter, 79117 Freiburg (DE)
(74) Vertreter: Dennemeyer & Associates S.A.
(86) Internationale Anmeldenummer: PCT/EP2018/059602
(87) Internationale Veröffentlichungsnummer: WO 2018/189398

(56) Entgegenhaltungen:
- WO-A1-02/072423
- WO-A1-2010/114842
- WO-A1-2011/140071
- WO-A2-2007/133590
- DE-A1-102006 030 068

## Beschreibung

Die Erfindung betrifft eine Vorrichtung zur Prozessierung einer flüssigen Probe nach dem Oberbegriff des Anspruchs 1.

Darüber hinaus betrifft die Erfindung ein Verfahren zur Prozessierung einer flüssigen Probe.

Aus dem Stand der Technik ist bekannt, dass Mikrotiterplatten oder ähnliche Testträger eine Vielzahl von Behältnissen aufweisen, in denen flüssige Proben, wie beispielsweise Zellkulturen, biologische Gewebe, DNA-Proben, Wirkstoffkandidaten, etc., enthalten sind. Die einzelnen flüssigen Proben werden üblicherweise mit Hilfe von Pipetten, Laborrobotern und/oder peripheren Laborgeräten, wie z.B. Inkubatoren, Reader, Schüttler, etc., manuell oder automatisiert prozessiert, um chemische und/oder biochemische Laborprozesse, wie z.B. Amplifikation bzw. Aufreinigung von DNA, Kultivierung von Zellen, Durchführen von Toxizitätsstudien an Zellkulturen, Analyse der Proteinexpression von Zellkulturen, Nachweis von Protein-Protein Interaktionen, Durchführen von Immunoassays, etc., zu realisieren.

Zur Durchführung der vielfältigen Operationen an den in den Testträgern vorgehaltenen, flüssigen Proben, welche für eine spezifische Anwendung erforderlich sind, existieren eine Fülle von Laborgeräten. Die Laborgeräte sind in der Lage, einzelne oder mehrere Prozessierungsschritte an den flüssigen Proben vorzunehmen. In den Laboren existiert üblicherweise eine Infrastruktur, welche darauf ausgelegt ist, in standardisierten Mikrotiterplatten bevorratete flüssige Proben von einer Prozessierungsstation zur nächsten zu transportieren, um einen kompletten Prozessierungsablauf zur realisieren.

Es sind eine Vielzahl von Laborgeräten und Verfahren bekannt, mit denen Mikrotiterplatten oder vergleichbare Träger prozessiert werden können. Den meisten Laborgeräten ist gemein, dass sie für die Bearbeitung einer oder mehrerer Mikrotiterplatten ausgelegt sind. Viele Laborgeräte haben auch mehrere Funktionen, um ggf. eine ganze Analyse oder zumindest Teile eines Gesamtprozesses vollautomatisiert durchführen zu können. Kann ein Prozess nicht in einem Laborgerät komplett durchgeführt werden, so werden typischerweise verschiedene Laborgeräte mit Hilfe von Automatisierungslösungen zu einer Anlage kombiniert, indem die Mikrotiterplatten von einer Prozessierungsstation zur nächsten transportiert werden.

Dieses Konzept führt jedoch zu teilweise sehr großen und teuren Anlagen, die gegebenenfalls nur gering ausgelastet sind. Insbesondere sind oftmals viele Automatisierungskomponenten, wie z.B. x-y-z-Positionierungssysteme oder automatische Pipettierstationen in vielen Anlagen mehrfach vorhanden, weil sie in verschiedenen Laborgeräten, wie beispielsweise des Transportsystems für die Mikrotiterplatte von Prozessierungsstation zu Prozessierungsstation, dem Achssystem des Pipettierroboters, dem Positioniersystem des Mikrotiter-Readers, etc., vorkommen. Diese Redundanz wird in der Praxis nicht benötigt.

Andererseits wird von den Anlagen der erforderliche Durchsatz unter Umständen nicht bewältigt, da einige Prozessierungsstationen zum Engpass werden können. Diese Engpässe können nicht einfach überwunden werden, da eine Skalierung der Anlage aufgrund der geringen Modularität und der oftmals linearen Prozessierung nicht ohne weiteres möglich ist. Aufgrund der konventionellen Automatisierung ist es außerdem kaum möglich, die verwendeten Flüssigkeitsmengen zu miniaturisieren und die Prozesse dadurch ressourcensparender, effizienter und schneller durchzuführen.

Als Alternative zu den eingesetzten Anlagen existieren Ansätze zur Prozessierung von Flüssigkeiten in sehr kleinen Mengen in geschlossenen mikrofluidischen Systemen. Diese Systeme sind auch bekannt unter den engl. Begriffen "Lab-on-a-Chip" oder "Micro-Total-Analysis-Systems". Diese Systeme sind sehr schnell und effizient, weisen jedoch bezüglich der vollständigen Automatisierung und bezüglich der Schnittstellen zu konventionellen Laborgeräten noch große Schwächen auf. Lab-on-a-Chip Systeme können aufgrund ihrer geschlossenen Testträger und der fest vorgegebenen Struktur der Prozessierung, oftmals nicht flexibel mit anderen vor- bzw. - nachgelagerten Prozessen kombiniert werden. Des Weiteren sind bei Lab-on-a Chip Systemen auch oftmals nur die Testträger und Flüssigkeitsmengen vergleichsweise klein, die Prozessierungs- oder Analysegeräte, welche diese Testträger prozessieren aber nicht.

Bei den bekannten Anlagen besteht somit einerseits ein hoher Standardisierungs- und Automatisierungsgrad mit hoher Flexibilität bei der Verwendung von Mikrotiterplatten und ähnlichen Trägern. Die Anlagen weisen jedoch den Nachteil einer großen, teuren und oftmals nicht optimal genutzten Laborinfrastruktur und eingeschränktem Miniaturisierungs- bzw. Kosteneinsparungspotential auf. Auf der anderen Seite besteht der Nachteil von stark miniaturisierten und hoch integrierten mikrofluidischen Lab-on-Chip-Systemen in deren geringer Flexibilität hinsichtlich der durchführbaren Prozesse, der Komplexität der Testträger, welche oftmals aufwändige, mikrofluidische Strukturen enthalten und der schwierigen Integration in vorgelagert oder nachgelagerte Prozessketten konventioneller Art.

WO 2007/133 590 A2 offenbart ein System, das eine optische Einheit mit einem Controller-Chip aufweist. Der Controller-Chip hat mehrere Reaktionszellen zur gleichzeitigen Reaktion von Katalysatorproben zur optischen Überwachung einer kinetischen Aktivität innerhalb jeder der Reaktionszellen.

DE 10 2006 030 068 A1 offenbart ein Verfahren zur Zu- und Abfuhr von Flüssigkeiten in Mikroreaktorenarrays durch einen oder mehrere Fluidkanäle, die in jeden einzelnen Mikroreaktor führen und individuell gesteuert und geregelt werden können. Die zu- oder abgeführten Flüssigkeitsmengen können während eines kontinuierlichen Schüttelvorgangs in das Volumen der Reaktionsflüssigkeit ein- oder abgeleitet und durch das Schütteln jeweils gleichmäßig gemischt werden.

Daher besteht die Aufgabe der Erfindung darin, eine Vorrichtung anzugeben, die den zur Prozessierung benötigten apparativen Aufbau verringert, klein und einfach ausgebildet ist, flexibel einsetzbar ist und einfach in die bestehende Laborumgebung integriert werden kann.

Diese Aufgabe wird durch eine Vorrichtung nach Anspruch 1 gelöst.

Darüber hinaus besteht eine Aufgabe der Erfindung darin, ein Verfahren anzugeben, mittels dem die Prozessierung einfacher und schneller durchgeführt werden kann.

Diese Aufgabe wird durch ein Verfahren nach Anspruch 10 gelöst.

Die erfindungsgemäße Vorrichtung weist den Vorteil auf, dass infolge der gestapelten Bauweise der Bestandteile der Vorrichtung eine kleine und einfach aufgebaute Vorrichtung bereitgestellt werden kann, mittels der die flüssige Probe prozessiert werden kann. Dabei können mit der Vorrichtung vorteilhafterweise die einzelnen Prozessierungsschritte direkt in oder an der Vorrichtung, insbesondere direkt im Träger, durchgeführt werden, was den Prozessierungsablauf vereinfacht und beschleunigt. Im Ergebnis wird durch die Betätigungseinrichtung und die Prozesseinrichtung eine miniaturisierte, automatisierte gleichzeitige Prozessierung von einer Vielzahl von flüssigen Proben, die in dem Träger enthalten sind, möglich. Insbesondere können mehrere Träger parallel in unterschiedlichen Vorrichtungen bearbeitet werden. Dadurch kommt es entgegen den bekannten Ausführungen zu keinen Engpässen mehr im Prozessablauf und/oder den Prozessierungsstationen, da die Träger nicht mehr in die Prozessierungsstationen eingebracht werden müssen.

Ein weiterer Vorteil ist, dass die Betätigungseinrichtung und die Prozessiereinheit sowohl einzeln, als auch zusammen mit dem Träger manuell oder automatisiert gehandhabt werden kann. Insbesondere besteht die Möglichkeit, die Vorrichtung manuell zusammenzubauen, indem die Betätigungseinrichtung und die Prozessiereinheit mit der Hand auf den Träger aufgesetzt werden, während hingegen die anschließende Prozessierung der flüssigen Probe automatisch erfolgt. Da die Prozessierung der flüssigen Probe automatisch erfolgt, besteht auch keine Notwendigkeit, separate Laborgeräte für beispielsweise die Handhabung der Prozessiereinheit anzuschaffen. Die Prozessiereinheit und der Träger können durch die konventionelle Automatisierungstechnik unter Verwendung der bestehenden Laborgeräte automatisch gehandhabt werden, insbesondere dann, wenn die Betätigungseinrichtung, die Prozessiereinheit und der Träger standardisierte Abmessungen haben, die z.B. den Außenmaßen von Mikrotiterplatten entsprechen. Sofern keine Automatisierungstechnik vorhanden ist, können die zuvor genannten Bauteile durch Labormitarbeiter gehandhabt werden.

Eine Integration in den konventionellen Prozessablauf ist möglich, weil als Träger aus dem Stand der Technik bekannte und genormte Träger, wie beispielsweise Mikrotiterplatten eingesetzt werden. Darüber hinaus bleibt aufgrund des Einsatzes des zuvor genannten Trägers auch die Kompatibilität mit konventionellen Laborgeräten erhalten. Sowohl etablierte Logistik-Systeme für die Träger, wie beispielsweise Mikrotiterplatten, als auch komplexe Analysegeräte, deren Miniaturisierung nicht möglich ist, können weiter verwendet werden.

Ein besonderer Vorteil der Vorrichtung besteht außerdem darin, dass die Vorrichtung, die die Betätigungseinrichtung, die Prozessiereinheit und den Träger aufweist, einem kleinen Laborgerät entspricht, das die Prozessierung durchführen kann. Es besteht somit nicht die Notwendigkeit eine Prozessierungsstation vorzusehen, die einen oder mehrere Prozessierungsschritte durchführen kann. Mittels der Vorrichtung kann für jeden Träger individuell der für ihn benötigte Prozessierungsschritt oder Prozessierungsschritte bereitgestellt werden. Dabei kann eine zeitlich lückenlose Prozessierung jedes einzelnen Trägers ermöglicht werden und Wartezeiten und Lagerung der Träger können aufgrund der individuellen Prozessierung der Träger in der Vorrichtung vermieden werden.

Die Miniaturisierung der Prozessierung oder einzelner Prozessierungsschritte ergibt sich, weil die Prozessiereinheit dazu ausgebildet und bestimmt ist, wenigstens einen Prozessierungsschritt durchzuführen. Dies bedeutet, dass die Prozessierung der flüssigen Probe von den großen Prozessierungsstationen hin zu der Prozessiereinheit der Vorrichtung verlagert wird. Dadurch lassen sich die Prozessierungsschritte effizienter und somit kostengünstiger durchführen. Insbesondere wird die Parallelisierbarkeit und Skalierbarkeit der Laborprozesse durch die Modularisierung der einzelnen Prozessschritte in einzelnen Prozessiereinheiten und Betätigungseinrichtungen mit spezifischer Funktion erheblich verbessert. Dadurch werden effizientere Automatisierungskonzepte für komplexe Aufgaben ermöglicht.

Als Prozessierung von Proben wird sowohl die verfahrenstechnische Prozessierung verstanden, als auch die im Rahmen von biochemischen Analysen oftmals erforderliche Detektion von biologischen und/oder chemischen und/oder physikalischen Eigenschaften der Probe. Dementsprechend kommen als Prozessierungsschritte das Zuführen oder Entnehmen von Fluid, das Durchmischen von Fluiden und/oder das Bewegen von Mikropartikeln in Betracht. Darüber hinaus handelt es sich bei dem Erfassen von optischen Eigenschaften eines Fluids, insbesondere unter Erzeugen von optischen Abbildungen, dem Heizen oder Kühlen des Fluids und/oder dem Messen von physikalischen Eigenschaften des Fluids, wie beispielsweise des pH-Werts und/oder der Sauerstoffkonzentration des Fluids und/oder der Fluoreszenzintensität des Fluids um Prozessierungsschritte. Natürlich sind noch weitere Prozessierungsschritte denkbar. Dabei kann die Prozessiereinheit einen oder mehrere der zuvor genannten Prozessierungsschritte durchführen.

Die Prozessierung der flüssigen Probe kann zum Zwecke von biologischen und/oder chemischen Preparation und/oder Reaktionen und/oder Analysen durchgeführt werden. Die flüssige Probe kann eine flüssige biologische oder chemische Probe sein. Insbesondere kann die flüssige Probe Zellen aufweisen, die in einer Flüssigkeit schwimmen. Das Behältnis des Trägers kann ein Mikrobioreaktor sein. In einem Mikrobioreaktor können zum Prozessieren der Probe bestimmte chemische und/oder biologische Reaktionen unter definierten Bedingungen ablaufen, wobei die Reaktionen unter anderem durch Zugabe und/oder Abfuhr von Fluiden steuerbar oder regelbar sind. Insbesondere können in dem Mikrobioreaktor beispielsweise Zellen kultiviert werden.

Das Fluid kann ein Gas oder eine Flüssigkeit, insbesondere die flüssige Probe, sein und ist beweglich und kann daher durch Pumpen, Ventile, Fluidleitungen, etc., geleitet und transportiert werden. Eine fluidische Verbindung zwischen zwei Bauteilen besteht, wenn das Fluid von einem Bauteil in das andere Bauteile strömen kann. Als Durchmischen der flüssigen Probe wird ein Vorgang verstanden, bei denen die Bestandteile der flüssigen Probe derart relativ zueinander bewegt werden, dass ein neues Anordnungsschema entsteht.

Bei einer besonderen Ausführung kann die Vorrichtung derart ausgebildet sein, dass die Prozessierung der flüssigen Probe in der Vorrichtung autonom erfolgt. Somit weist die Vorrichtung keine Anschlüsse auf, mittels der ein Prozessierungsmedium in die Vorrichtung eingebracht wird. Dies bedeutet, dass die Vorrichtung die flüssige Probe selbsttätig prozessieren kann, unabhängig davon, wo die Vorrichtung angeordnet ist. Dies bietet den signifikanten Vorteil, dass Vorrichtungen für die Prozessierung der flüssigen Probe an beliebigen Orten abgelegt werden können und somit keine Engpässe in den Prozessierungsstationen entstehen, weil die Vorrichtung zur Prozessierung der flüssigen Probe nicht in die Prozessierungsstation eingebracht werden muss.

Die Vorrichtung kann ein Gehäuse aufweisen, das mit dem Träger wieder lösbar verbunden ist und den Träger abschließt. Insbesondere kann die Betätigungseinrichtung zwischen dem Gehäuse und dem Träger angeordnet sein. Dabei kann das Gehäuse, insbesondere unmittelbar, auf die Betätigungseinrichtung gesetzt werden. Das Gehäuse bietet den Vorteil, dass die Betätigungsvorrichtung, die Prozessiereinheit und der Träger von äußeren Einflüssen geschützt sind.

Dabei kann das Gehäuse und/oder die Betätigungseinrichtung und/oder die Prozessiereinheit derart ausgebildet sind, dass sie durch dieselbe, insbesondere mechanische, Handhabungsvorrichtung handhabbar sind wie der Träger. Insbesondere kann die Handhabungsvorrichtung ein Roboter sein, der zum Handhaben der zuvor genannten Bauteile eingesetzt wird. Da das Gehäuse, die Betätigungseinrichtung und die Prozessiereinheit die gleichen Außenmaße wie der Träger aufweisen kann die konventionelle Automatisierungs- und Logistik-Infrastruktur, welche auf Träger ausgerichtet ist, weiter genutzt werden. Dies unterscheidet die Vorrichtung wesentlich von den bekannten Ausführungen, bei denen die konventionelle Automatisierungs- und Logistik-Infrastruktur nicht mehr genutzt werden kann.

Die Betätigungseinrichtung kann mit der Prozessiereinheit wieder lösbar verbunden sein. Darüber hinaus kann die Prozessiereinheit mit dem Träger wieder lösbar verbunden sein und/oder unmittelbar auf den Träger aufgesetzt sein. Außerdem kann das Gehäuse wieder lösbar oder fest mit der Betätigungseinrichtung verbunden sein. Das Verbinden der einzelnen Bauteile miteinander kann manuell oder automatisch erfolgen. Infolge der einfachen Abnehmbarkeit des Gehäuses, der Prozessiereinheit und der Betätigungseinrichtung von dem Träger bleibt die Kompatibilität des Trägers zur übrigen Laborgeräteinfrastruktur und die Möglichkeit der Nutzung vorhandener, stationärer, komplexer Prozessierungs- und Analysegeräte erhalten.

Bei einer besonderen Ausführung kann das Gehäuse eine Eingabevorrichtung, insbesondere ein Touchdisplay, zum Eingeben von Daten an die, insbesondere in die Betätigungseinrichtung integrierte, elektrische Steuer- oder Regeleinheit aufweisen. Dadurch kann der Benutzer auf besonders einfache Weise die elektrische Steuer- oder Regeleinheit programmieren und/oder den Prozessvorgang starten und/oder Prozessparameter eingeben.

Das Gehäuse kann kastenartig ausgeführt sein, wobei die Betätigungseinrichtung, insbesondere die elektrische Steuer- oder Regeleinheit, und/oder die Prozessiereinheit und/oder der Träger, insbesondere ein Teil des Trägers, in einem Hohlraum des Gehäuses angeordnet sind. Dabei kann ein Trägerboden die Vorrichtung zu einer Seite hin abschließen. Dies bedeutet, dass die Außenkontur der Vorrichtung durch das Gehäuse und/oder den Trägerboden begrenzt sein können.

Die Betätigungseinrichtung kann einen, insbesondere wieder aufladbaren, elektrischen Speicher aufweisen, der die elektrische Steuer- oder Regeleinheit mit elektrischer Energie versorgt.

Natürlich kann der elektrische Speicher auch weitere Bauteile der Vorrichtung mit elektrischer Energie versorgen. Der elektrische Speicher dient somit zum Versorgen der elektrischen Steuer-oder Regeleinheit und/oder weiterer Bauteile mit elektrischer Energie. Darüber hinaus kann die Betätigungseinrichtung ein Kommunikationsmittel zum, insbesondere kabellosen, Versenden und/oder Empfangen von Daten aufweist. Insbesondere kann das Kommunikationsmittel eine Kommunikation über WLAN, Bluetooth, etc. realisiert werden. Die Vorrichtung kann somit autonom mit ihrer Laborumgebung kommunizieren, um durch sie gewonnene Daten zu übertragen und Prozessanweisungen zu erhalten. Somit sind die durch die Betätigungseinrichtung und die Prozessiereinheit abgebildeten (Teil-) Prozesse einfach parallelisierbar, skalierbar und dennoch voll in den konventionellen Prozessablauf integrierbar. Außerdem kann die Betätigungseinrichtung mittels des Kommunikationsmittels mit einer nachstehend näher beschriebenen weiteren Betätigungseinrichtung kommunizieren.

Darüber hinaus kann die Betätigungseinrichtung wenigstens eine Pumpe aufweisen, die durch die elektrische Steuer- oder Regeleinheit ansteuerbar ist. Die Pumpe bietet den Vorteil, dass sie insbesondere bei Prozessiereinheiten, die eine mikrofluidische Struktur aufweisen, als Antrieb fungieren kann, damit der Prozessierungsschritt durchgeführt werden kann. Das Vorsehen der Pumpe und/oder eines anderen Antriebs zum Antreiben der Prozessiereinheit in der Betätigungseinrichtung bietet den Vorteil, dass die Prozessiereinheit und die Betätigungseinrichtung ein kompaktes und unabhängiges Laborgerät bilden, welches nicht wesentlich größer als der Träger selbst ist und seine Aufgabe autonom und effizient durchführen kann.

Darüber hinaus kann die Betätigungsvorrichtung wenigstens einen Tank zur Lagerung eines Prozessierungsmediums aufweisen. Das Prozessierungsmedium kann ein Feststoff, eine Flüssigkeit oder ein Gas sein. Der Tank kann mit der Pumpe und/oder der Prozessiereinheit fluidisch verbunden sein. Insbesondere kann die Pumpe das in dem Tank befindliche Prozessierungsmedium in die Prozessiereinheit fördern. Alternativ oder zusätzlich kann der Tank mittels eines Ventils mit der Prozessiereinheit fluidisch verbunden sein. Dies bietet sich insbesondere dann an, wenn das Prozessierungsmedium ein Gas ist, das unter hohem Druck im Tank gelagert ist. Die elektrische Steuer- oder Regeleinheit kann das Ventil, insbesondere eine Ventilstellung, steuern.

Außerdem kann die Betätigungsvorrichtung eine Messeinheit, insbesondere einen Drucksensor, aufweisen, der mit der elektrischen Steuer- oder Regeleinheit elektrisch verbunden ist und/oder mit der Prozessiereinheit fluidisch verbunden ist. Dabei kann die elektrische Steuer- oder Regeleinheit die Pumpe anhand der von der Messeinheit übermittelten Daten steuern oder regeln.

Die Betätigungseinrichtung kann eine Platte aufweisen, auf der die elektrische Steuer- oder Regeleinheit und/oder der elektrische Speicher und/oder das Kommunikationsmittel und/oder die Pumpe und/oder der Tank und/oder die Messeinheit angeordnet sind. Durch das Anordnen der Bauteile auf der Platte wird eine kompakt ausgeführte Betätigungseinrichtung realisiert. Die Platte kann derart ausgeführt sein, dass sie die Prozessiereinheit abdeckt.

Bei dem, insbesondere mittelbaren oder unmittelbaren, Aufsetzen der Betätigungseinrichtung auf die Prozessiereinheit kann automatisch und/oder unmittelbar eine, insbesondere fluidische oder elektrische, Verbindung zwischen der Betätigungseinrichtung und der Prozessiereinheit realisiert werden. Dadurch lässt sich eine Verbindung zwischen der Betätigungseinrichtung und der Prozessiereinheit auf einfache Weise herstellen, ohne das ein Eingriff seitens eines Labormitarbeiters oder einer Handhabungseinrichtung notwendig ist.

Darüber hinaus kann, insbesondere die Vorrichtung derart ausgebildet sein, dass, bei einem Aufsetzen der Betätigungseinrichtung auf die Prozessiereinheit oder bei einem Abnehmen der Betätigungseinrichtung von der Prozessiereinheit das in der Prozessiereinheit befindliche Prozessierungsmedium und/oder der in der Prozessiereinheit befindliche Teil der flüssigen Probe nicht in Kontakt mit der Betätigungseinrichtung gelangt. So kann die Prozessiereinheit einen Filter aufweisen, der flüssigkeitsundurchlässig ist, so dass eine Kontamination der Betätigungseinrichtung durch das Prozessierungsmedium und/oder die flüssige Probe auf einfache Weise verhindert werden kann.

Die Betätigungseinrichtung kann schlauchlos oder kabellos mit der Prozessiereinheit verbunden ist. Insofern kann auch eine kompakte Verbindung zwischen der Betätigungseinrichtung und der Prozessiereinheit realisiert werden. Die Betätigungseinrichtung kann derart ausgeführt und dazu bestimmt sein, dass sie nur mit Prozessiereinheiten verbunden werden kann, die einen bestimmten Prozessierungsschritt oder mehrere bestimmte Prozessierungsschritte durchführen können.

Ein Zwischenelement kann zwischen der Betätigungseinrichtung und der Prozessiereinheit angeordnet sein. Insbesondere können die Betätigungseinrichtung und die Prozessiereinheit jeweils unmittelbar mit dem Zwischenelement verbunden sein. Das Zwischenelement kann als separates Bauteil ausgeführt sein, das mit der Betätigungseinrichtung und mit der Prozessiereinheit, insbesondere mechanisch, verbunden ist. Bei dieser Ausführung kann das Zwischenelement wenigstens eine Schnittstelle aufweisen, mittels der eine fluidische Verbindung der Betätigungseinrichtung mit der Prozessiereinheit ermöglicht wird.

Alternativ kann mittels des Zwischenelements eine stoffschlüssige Verbindung zwischen der Betätigungseinrichtung und der Prozessiereinheit realisiert werden. Bei dieser Ausführung kann das Zwischenelement ein Kleber sein. Dabei können nur die Ränder der Betätigungseinrichtung und der Prozessiereinheit mittels des Zwischenelements miteinander verklebt werden. Auch bei dieser Ausführung kann eine fluidische Verbindung zwischen der Betätigungseinrichtung und der Prozessiereinheit bestehen.

Bei einer besonderen Ausführung kann die die Prozessiereinheit wenigstens einen weiteren Tank zur Lagerung eines weiteren Prozessierungsmediums aufweisen. Das Vorsehen eines weiteren Tanks bietet den Vorteil, dass keine Anschlüsse zu Reservoirs notwendig sind, die das Prozessierungsmedium enthalten und außerhalb der Vorrichtung angeordnet sind. Das weitere Prozessierungsmedium kann ein Feststoff, eine Flüssigkeit oder ein Gas sein. Im Ergebnis kann eine autonome Prozessierung der flüssigen Probe in der Vorrichtung erfolgen.

Die Prozessiereinheit kann als Einwegbauteil ausgeführt sein. Darüber hinaus kann die Prozessiereinheit derart ausgebildet sein, dass sie bei einem Aufsetzen auf den Träger, das Behältnis oder die Behältnisse dichtend abschließt. Dazu kann die Prozessiereinheit eine Dichtung aufweisen. Dadurch kann verhindert werden, dass beispielsweise bei einem Durchmischen der flüssigen Probe in dem Behältnis die flüssige Probe aus dem Behältnis herausschwappt und/oder die flüssige Probe verdunstet.

Die Vorrichtung kann auch eine weitere Prozessiereinheit aufweisen. Die weitere Prozessiereinheit dient zum Durchführen wenigstens eines weiteren Prozessierungsschritts. Insbesondere kann die weitere Prozessiereinheit einen oder mehrere der oben genannten Prozessierungsschritte durchführen. Darüber hinaus kann die Vorrichtung eine weitere Betätigungseinrichtung aufweisen, wobei die weitere Prozessiereinheit und die Betätigungseinrichtung an der von der Prozessiereinheit abgewandten Seite des Trägers angeordnet sein können.

Insbesondere kann die weitere Prozessiereinheit zwischen dem Träger und der weiteren Betätigungseinrichtung angeordnet sein. Dabei kann der Träger auf die weitere Prozessiereinheit, insbesondere unmittelbar, aufgesetzt sein. Die weitere Prozessiereinheit kann auf die weitere Betätigungseinrichtung, insbesondere unmittelbar, aufgesetzt sein. Die weitere Prozessiereinheit kann wenigstens eine weitere optische Erfassungseinrichtung zum Erfassen einer Eigenschaft einer flüssigen Probe aufweisen. Die optische Erfassungseinrichtung kann eine optische Abbildungsvorrichtung, wie beispielsweise eine Kamera aufweisen. Mittels der optischen Abbildungsvorrichtung kann eine Abbildung der flüssigen Probe und/oder eines Sensors erzeugt werden. Dazu kann das Behältnis, insbesondere der Behältnisboden, transparent sein.

Bei einem Träger, der mehrere Behältnisse aufweist, kann die weitere optische Erfassungseinrichtung mehrere optische Abbildungsvorrichtungen aufweisen. Dabei entspricht die Anzahl der optischen Abbildungsvorrichtungen der Anzahl der Behältnisse, sodass zu jeder flüssigen Probe eine Abbildung erstellt werden kann.

Dabei können die weitere Prozessiereinheit und die Betätigungseinrichtung in einem weiteren Hohlraum eines weiteren Gehäuses angeordnet sein, wobei das weitere Gehäuse mit dem Träger wieder lösbar verbindbar ist. Die weitere Betätigungseinrichtung ist, insbesondere unmittelbar, auf das weitere Gehäuse gesetzt. Das weitere Gehäuse schützt die weitere Prozessiereinheit und die weitere Betätigungseinrichtung von äußeren Einflüssen.

Die weitere Betätigungseinrichtung und die Betätigungseinrichtung können datentechnisch kommunizieren. Dies ist insbesondere dann vorteilhaft, wenn die durch die weitere optische Erfassungseinrichtung erfassten Eigenschaften der flüssigen Probe zur Regelung der Prozessierung der flüssigen Probe eingesetzt werden. Das Vorsehen der weiteren Prozessiereinheit bietet somit den Vorteil, dass eine, insbesondere selbsttätige, Überwachung des Behältnisses, insbesondere der flüssigen Probe durch die Vorrichtung möglich ist. Dabei kann bei der Überwachung eine Eigenschaft der flüssigen Probe optisch erfasst werden und die elektrische Steuer- oder Regeleinheit kann den durch die Prozessiereinheit durchgeführten Prozessierungsschritt abhängig von der erfassten Eigenschaft regeln.

Der Träger kann mehrere Behältnisse aufweisen. Insbesondere kann der Träger eine Mikrotiterplatte sein. Die Mikrotiterplatte kann eine Platte mit 6 oder 24 oder 96 oder 384 oder 1536 oder 3456 oder 6144 Behältnissen sein. Dabei kann die Mikrotiterplatte eine rechteckige Platte sein und/oder aus Kunststoff bestehen. Die voneinander isolierten Behältnisse können in Reihen und Spalten angeordnet sein. In den einzelnen Behältnissen können unterschiedliche flüssige Proben enthalten sein.

Der Träger ist derart ausgeführt, dass bei einem Entfernen der Aufsatzvorrichtung die Behältnisse untereinander nicht fluidisch verbunden sind. Insbesondere sind keine Fluidleitungen in Wänden des Trägers vorhanden, über die wenigstens zwei Behältnisse fluidisch miteinander verbunden sind.

Die Prozessiereinheit, insbesondere gemäß einer ersten Variante, kann wenigstens eine Fluidleitung aufweisen. Darüber hinaus kann die Prozessiereinheit einen Aufsatz aufweisen, von dem sich die Fluidleitung erstreckt. Die Fluidleitung ist derart ausgeführt und dazu bestimmt, dass sie in die flüssige Probe ragt. Die Fluidleitung kann rigid ausgeführt sein. Insbesondere kann die Fluidleitung eine Kanüle sein.

Der Aufsatz kann wenigstens einen Fluidkanal aufweisen. Wenigstens eine Fluidleitung der Prozessiereinheit kann mit dem Fluidkanal fluidisch verbunden sein. Darüber hinaus kann die Prozessiereinheit eine andere Fluidleitung aufweisen. Der Aufsatz kann einen anderen Fluidkanal aufweisen. Die andere Fluidleitung kann mit dem anderen Fluidkanal fluidisch verbunden sein. Dabei können der Fluidkanal und der andere Fluidkanal nicht miteinander fluidisch verbunden sein. Dies kann dadurch realisiert werden, dass der Fluidkanal und der andere Fluidkanal im Aufsatz in unterschiedlichen Ebenen verlaufen. Dabei können sich die Fluidkanäle innerhalb des Aufsatzes auch kreuzen.

Der Fluidkanal kann mit der Pumpe fluidisch verbunden sein. Darüber hinaus kann der Fluidkanal mit dem Tank und/oder mit dem weiteren Tank fluidisch verbunden sein. Der andere Fluidkanal kann mit einer anderen Pumpe fluidisch verbunden sein. Darüber hinaus kann der andere Fluidkanal mit dem Tank und/oder mit dem weiteren Tank fluidisch verbunden sein.

Die Prozessiereinheit kann wenigstens ein weiteres Ventil aufweisen. Mittels des weiteren Ventils kann ein Strömen des Prozessierungsmediums und/oder des weiteren Prozessierungsmediums und/oder der flüssigen Probe in der Prozessiereinheit gesteuert werden, wobei das weitere Ventil mit der elektrischen Steuer- oder Regeleinheit verbunden ist. Insbesondere kann jeder Fluidleitung jeweils ein weiteres Ventil zugeordnet sein. Dadurch lässt sich der Fluidstrom innerhalb der Prozessiereinheit auf besonders einfache Weise, insbesondere mittels der elektrischen Steuer- oder Regeleinheit, steuern. Dadurch können Kosten gespart werden, weil kein Prozessierungsmedium mehr verschwendet wird.

Wie oben bereits beschrieben kann die Vorrichtung die Prozessierung der flüssigen Probe autonom durchführen. Dies ist auf ganz besonders einfache Weise möglich, wenn die Vorrichtung die Prozessiereinheit gemäß der ersten Variante aufweist. So entfällt hier die Notwendigkeit, extern angeschlossene Fluidreservoire, die über Schläuche angeschlossen werden, sowie externe Pumpen, Ventile und externe Steuerungen für die Prozessierung vorzusehen.

Mittels der zuvor beschriebenen Prozessiereinheit gemäß der ersten Variante lassen sich eine Vielzahl von Prozessierungsschritten und/oder Betriebsweisen der Vorrichtung realisieren. Die nachfolgend beschriebenen Betriebsweisen sind möglich, weil die Prozessiereinheit die zuvor beschriebene mikrofluidische Struktur aufweist und/oder die wenigstens eine Fluidleitung zumindest in das Behältnis eindringt, insbesondere in die flüssige Probe ragt.

So kann bei einer ersten Betriebsweise mittels der Prozesseinheit wahlweise ein Durchmischen der in einem Behältnis des Trägers befindlichen flüssigen Probe oder ein Einsaugen eines Teils der flüssigen Probe oder ein Dispensieren eines Fluids, insbesondere des zuvor eingesaugten Teils der flüssigen Probe oder eines Gases, in die flüssige Probe durchgeführt werden. Somit können mittels der Prozessiereinheit eine Vielzahl von Prozessierungsschritten durchgeführt werden.

Dabei kann das Durchmischen der flüssigen Probe dadurch durchgeführt wird, dass abwechselnd und/oder mehrmals hintereinander ein Teil der in dem Behältnis befindlichen flüssigen Probe in die Fluidleitung der Prozessiereinheit eingesaugt und anschließend der in die Fluidleitung eingesaugte Teil der flüssigen Probe unmittelbar in die flüssige Probe dispensiert wird. Alternativ oder zusätzlich kann ein Durchmischen der flüssigen Probe dadurch erreicht werden, dass an einem Auslass der Fluidleitung eine Gasblase erzeugt wird und abwechselnd und/oder mehrmals hintereinander ein Gasblasendurchmesser vergrößert und verkleinert wird.

Die erste Betriebsweise der Vorrichtung ist besonders im Bereich der biotechnologischen Produktion von Vorteil. So wird die Herstellung von Wirkstoffen, wie zum Beispiel Proteinen, Antikörpern, etc., in der biotechnologischen und pharmazeutischen Produktion zunehmend auf zellulärer Basis betrieben. Dazu werden durch Klonierung aus einzelnen, gentechnisch veränderten Zellen große, genetisch identische Zellpopulationen herangezogen. Diese wachsen und produzieren in Edelstahltanks den gewünschten Wirkstoff. Diese hochkomplexe Prozesskette beginnt mit einer Einzelzelle. Die Einzelelle wird nach der gentechnischen Veränderung in dem Behältnis des Trägers, wie beispielsweise einer Mikrotiterplatte, vermehrt.

Vor der Vereinzelung lebt die Zelle mit Millionen von gleichartigen Zellen in einer sog. Schüttelkultur. Dass heißt die Zellen werden unter ständigem Schütteln in einem Schwebezustand in ihrem flüssigen Medium aufbewahrt. Die Zelle ist Schütteln somit gewöhnt. Vereinzelt man sie in das Behältnis des Trägers so erfährt die Zelle fluidischen Stillstand, weil sich die aus dem Stand der Technik bekannten Mikrotiterplatten, die Behältnisse mit kleinem Volumen haben, nicht geschüttelt werden können. Die kleinen Behältnisse haben einen zu hohen Kapillardruck und zudem besteht die Gefahr, dass bei zu heftigem Schütteln die flüssige Probe über den Deckel hinausschwappt. Daher ist es üblich, derartige Mikrotiterplatten stillliegend zu lagern. Dies bedeutet Stress für die Zelle, denn ihre natürlichen Umgebungsbedingungen haben sich geändert. Zudem ist die Zelle isoliert von Artgenossen, was einen zusätzlichen Stressfaktor bedeutet.

Die erfindungsgemäße Vorrichtung kann die Schüttelbewegung durch den Vorgang des Einsaugens und Dispensierens und das damit resultierende Durchmischen der flüssigen Probe nachbilden, ohne dass die Notwendigkeit besteht, den Träger zu bewegen. Damit ist einer der beiden Stressfaktoren der Zellen minimiert oder nicht mehr vorhanden. Die Chancen, dass aus einer Zelle eine Kolonie heranwächst sind somit signifikant erhöht.

Bei einer zweiten Betriebsweise kann mittels der Prozessiereinheit wenigstens ein Gemisch erzeugt werden, das wenigstens zwei Prozessierungsmedien aufweist. Dabei kann, insbesondere zum Erzeugen des Gemisches, mittels der Prozessiereinheit ein Prozessierungsmedium mit einer vorgegebenen Menge in das Behältnis zugeführt und anschließend mittels der Prozessiereinheit ein anderes Prozessierungsmedium mit einer vorgegebenen Menge in dasselbe Behältnis zugeführt werden. Anschließend kann mittels der Prozessiereinheit das Prozessierungsmedium und das andere Prozessierungsmedium in ein anderes Behältnis zugeführt werden, wobei das in dem anderen Behältnis erzeugte andere Gemisch eine andere Menge an dem Prozessierungsmedium und/oder eine andere Menge an dem anderen Prozessierungsmedium aufweist als das in dem Behältnis befindliche Gemisch.

Im Ergebnis kann mittels der zuvor beschriebenen zweiten Betriebsweise eine Verdünnungsreihe, die z.B. bei einem Proteinsynthese oder einem Wirkstoffscreening verwendet wird, erzeugt werden. Durch die gezielte Beförderung von definierten Fluidvolumina in einzelne Behältnisse lassen sich in den Behältnissen Gemische mit unterschiedlichen Konzentrationen erzeugen. Je nach Anzahl der in der Prozessiereinheit und/oder der Betätigungseinrichtung vorgelagerten Fluide lassen sich so innerhalb kürzester Zeit sogar komplexe, mehrstufige Verdünnungen erstellen.

Ganz besonders vorteilhaft ist die zweite Betriebsweise, wenn die Vorrichtung die oben beschriebene weitere Prozessiereinheit aufweist, die die weitere optische Erfassungseinrichtung zur Erfassung einer Eigenschaft der flüssigen Probe aufweist. Mittels der weiteren optischen Erfassungseinrichtung kann das Mischungsverhältnis in einem Behältnis überwacht werden. Somit ist die automatische Bestimmung des korrekten Mischungsverhältnisses pro Behältnis möglich. Im Ergebnis kann durch die weitere Prozessiereinheit sichergestellt werden, dass mittels der Prozessiereinheit exakte Mengen an Prozessierungsmedien in das jeweilige Behältnis zugeführt werden können, um das gewünschte Mischungsverhältnis in dem jeweiligen Behältnis zu erreichen.

Bei einer dritten Betriebsweise der Prozessiereinheit kann mittels der Prozessiereinheit eine flüssige Probe aus einem Behältnis in ein anderes Behältnis, insbesondere unter einer konstanten Flussrate, transferiert wird. Das Transferieren der flüssigen Probe kann zirkulierend durch die einzelnen Behälter des Trägers erfolgen. Alternativ kann das Transferieren der flüssigen Probe beendet werden, nachdem die flüssige Probe ausgehend von einem Startbehältnis in ein vorgegebenes Behältnis, insbesondere ein Endbehältnis, dispensiert wird. Durch die dritte Betriebsweise ist ein Durchmischen des Trägers mit verschiedenen Medien möglich.

Bei einer vierten Betriebsweise kann die Prozessiereinheit zur Kontaminationsbekämpfung eingesetzt werden. Dazu kann mittels der weiteren Prozessiereinheit überwacht werden, ob die flüssige Probe, insbesondere eine Zellkultur, mit einem Fremdkörper, wie beispielsweise Bakterien, Sporen oder Keimen, befallen ist. Sofern ein Befall der flüssigen Probe mit einem Fremdkörper mittels der weiteren Prozessiereinheit festgestellt wird, kann mittels der Prozessiereinheit ein Prozessierungsmedium, insbesondere ein Gegenmittel, wie beispielsweise Antibiotika, in das Behältnis mit befallenen flüssigen Proben eingebracht werden.

Die weitere optische Erfassungseinrichtung kann in vorgegebenen Zeitabständen, beispielsweise alle sechs Stunden, eine Abbildung der flüssigen Probe machen. Darüber hinaus kann die weitere optische Erfassungseinrichtung basierend aus der erzeugten Abbildung den Grad des Befalls bestimmen. Wird in einem Behältnis ein akuter Befall festgestellt, wird mittels der Prozessiereinheit ein Gegenmittel, insbesondere ein Antibiotikum, in ausschließlich das befallene Behältnis zugeführt. Die Vorrichtung kann sich das befallene Behältnis merken und kann es später melden, damit dieses aus den weiterführenden, pharmazeutischen Prozessen ausgeschlossen wird. Im Ergebnis ist nur ein Behältnis verloren, nicht aber mehr alle Behältnisses des Trägers.

Darüber hinaus kann die Prozessiereinheit gemäß der ersten Variante noch für eine Vielzahl von weiteren Applikationen genutzt werden. Insbesondere kann die Prozessiereinheit für Applikationen genutzt werden, bei denen ein Fluid oder mehrere Fluide in ein Behältnis oder in mehrere Behältnisse zugeführt werden müssen. Dazu zählen Assays, Screening, Proteinaufreinigung, Zellkultur, oder ganz allgemein das Mischen von Fluiden.

Die erfindungsgemäße Vorrichtung, die die Prozessiereinheit gemäß der ersten Variante aufweist, ist in der Lage, alle diese Operationen mit verschiedensten Medien autark durchzuführen. Um die Kontrolle und Rückführbarkeit der applizierten Schritte gewährleisten zu können, kann die Vorrichtung die weitere Prozessiereinheit aufweisen. Diese ermöglicht ein Auslesen der Zustände der flüssigen Probe und/oder von in der Vorrichtung befindlicher Sensoren beispielsweise über optische Messung wie Transmission, Chemilumineszenz oder Fluoreszenz.

Anders als in herkömmlichen Prozessen können all diese Schritte von der erfindungsgemäßen Vorrichtung in den abgeschlossenen Behältnissen des Trägers unter kontrollierten Bedingungen durchgeführt werden. Dabei ist bei der erfindungsgemäßen Vorrichtung im Vergleich zu den aus den Stand der Technik bekannten Vorrichtung die Prozessstabilität sehr viel höher, die Prozesse laufen homogener ab und es entfallen bisherig notwendige, nachteilige Schritte wie der Transfer zwischen verschiedenen Systemen und das Öffnen des Behältnisses, beispielsweise durch Abnahme des Deckels oder eines anderen Verschlusselements, was zu Verdunstung führt und Kontamination begünstigen kann.

Letzteres ist gerade bei kleinen Fluidmengen von wenigen Mikrolitern bis einigen Nanolitern ein zunehmendes Problem. Die sensiblen Reaktionsgemische funktionieren nur dann reproduzierbar, wenn die zugegebenen Prozessierungsmedien in ihrem Mischungsverhältnis exakt übereinstimmen. Je kleiner die Volumina desto größer wird der Einfluss von Verdunstung. Der Trend geht klar zu solch kleinen Volumina, denn kostbare und teure Prozessierungsmedien spielen in immer mehr Applikationen eine größere Rolle, wie z.B. bei der DNA- und RNA-Sequenzierung.

Die Prozessiereinheit gemäß der ersten Variante mit der mikrofluidischen Strukturen hat den Vorteil, dass sie nur geringe Totvolumina hat, was den Verlust an Prozessierungsmedien im Vergleich zu großen Pipettierrobotern minimiert. Die hermetisch dichtende Prozessiereinheit verbleibt während des gesamten Prozesses auf dem Träger und verhindert so effektiv Verdunstung.

Bei einer alternativen Prozessiereinheit, insbesondere gemäß einer zweiten Variante, kann die Prozessiereinheit ein Heizelement zum Heizen der flüssigen Probe und/oder ein Kühlelement zum Kühlen der flüssigen Probe aufweisen. Dabei kann die Prozessiereinheit einen weiteren Fluidkanal aufweisen, in dem ein Heizmittel oder ein Kühlmittel strömen kann. Das Heizmittel oder Kühlmittel kann innerhalb der Prozessiereinheit strömen und/oder ist mit der flüssigen Probe nicht fluidisch verbunden. Dabei kann die Prozessiereinheit einen Temperatursensor aufweisen, der die Temperatur des Heizelements und/oder Kühlelements und/oder des Heiz mittels und/oder Kühlmittels misst. Die elektrische Steuer- oder Regeleinheit kann mittels eines mit dem Temperatursensor ermittelten Werts das Heizen oder Kühlen der flüssigen Probe regeln.

Das Behältnis des Trägers kann in einer Aufnahme eines Aufnahmeelements angeordnet sein. Bei einer ganz besonders vorteilhaften Ausführung kann das Aufnahmeelement als Kühlelement dienen. Zum Kühlen der flüssigen Probe muss das Aufnahmeelement lediglich eine niedrigere Temperatur als die in dem Behältnis befindliche Probe aufweisen. Alternativ oder zusätzlich kann das Aufnahmeelement als thermisches Isolator wirken, der besonders ein Abkühlen der erhitzten flüssigen Probe verhindern soll.

Die Prozessiereinheit gemäß der zweiten Variante kann bei Untersuchungen zum Nachweis einer bestimmten Substanz (Assays) relevant sein. Bei vielen Assays ist die Temperatur der Probe entscheidend für die chemische Reaktion. Neben isothermen Assays, die eine möglichst konstante Temperatur erfordern, gibt es auch eine Vielzahl von Assays bei denen eine periodische Änderung der Temperatur notwendig ist.

Das berühmteste Beispiel ist hier wohl die sog. "Polymerase Chain Reaction (PCR)" zur Amplifikation von DNA. Herkömmlicherweise werden zur Durchführung solcher PCR-Assays Testträger in ein Laborgerät eingebracht, welches den gesamten Testträger, samt enthaltener Flüssigkeit, nach vorgegebenen Protokollen möglichst schnell auf die jeweils gewünscht Temperatur bringt. Mit Hilfe der Prozessiereinheit gemäß der zweiten Variante kann diese Funktion schneller, genauer und energiesparender durchgeführt werden. So kann die Prozessiereinheit hervorstehende Stege mit Heiz- und/oder Kühlelementen enthalten, welche in die Probe hineinreichen und diese direkt in der Flüssigkeit auf die gewünschte Temperatur bringen. Temperatursensoren können im Heiz- und/oder Kühlelement vorgesehen sein und somit eine präzise Regelung der Flüssigkeitstemperatur ermöglichen.

Insbesondere ist eine Vorrichtung von Vorteil, die die weitere Prozessiereinheit mit der weiteren optischen Erfassungseinrichtung aufweist. Mit Hilfe der weiteren optischen Erfassungseinrichtung, welche unterhalb des Trägers angebracht ist, kann die Fluoreszenzintensität im Behältnis während der Reaktion beobachtet werden. Somit können sogenannte "Real-Time-PCR"-Assays einfach durchgeführt werden.

Eine alternative Prozessiereinheit, insbesondere gemäß einer dritten Variante, kann eine Analyseeinheit zum Analysieren der flüssigen Probe aufweisen. Die Analyseeinheit kann wenigstens einen Sensor zum Erfassen einer Eigenschaft der flüssigen Probe aufweisen. Insbesondere kann die Analyseeinheit einen Biosensor und/oder einen Temperatursensor und/oder einen Sauerstoffsensor aufweisen. Der in die Prozessiereinheit eingesaugte Teil der flüssigen Probe kann zu jedem Sensor geleitet werden. Die einzelnen Sensoren können beispielsweise durch ein Fluoreszenzsignal ausgelesen werden, welches durch eine optische Erfassungseinrichtung, die in der Prozessiereinheit angeordnet sein kann, erfasst werden kann. Alternativ oder zusätzlich kann der Sensor durch die weitere optische Erfassungseinrichtung der weiteren Prozessiereinheit erfasst werden.

Darüber hinaus kann die Analyseeinheit eine Ausschusskammer zur Aufnahme der flüssigen Probe aufweisen, wobei die Ausschusskammer dem Sensor oder, insbesondere allen Sensoren, strömungstechnisch nachgeschaltet ist. Dabei kann der eingesaugte Teil der flüssigen Probe nach dem Analysieren in die Ausschusskammer geleitet werden. Dies bedeutet, dass der eingesaugte Teil der flüssigen Probe nicht wieder zurück in das Behältnis dispensiert wird.

Darüber hinaus kann die Prozessiereinheit auch eine mikrofluidische Struktur aufweisen, wie beispielsweise die Prozessiereinheit gemäß der ersten Variante. Infolge der mikrofluidischen Struktur ist es möglich, dass Prozessierungsmedien einzelnen Behältnissen des Trägers zugeführt werden, wenn die Analyse ergibt, dass ein Handlungsbedarf besteht.

Die Prozessiereinheit gemäß der dritten Variante kann besonders vorteilhaft im Zellbereich eingesetzt werden. So werden sehr sensible Zellen, wie beispielsweise Stammzellen, wie MSC oder iPS, in der modernen Medizin im Zuge von Therapien eingesetzt. Die Zellen reagieren extrem auf Stress, erzeugt durch die Änderung äußerer Einflüsse, wie beispielsweise dem Absinken der Temperatur, pH-Wert Schwankungen, Schwankungen im Kohlenstoffdioxid-Level und/oder UV-Licht. Dieser Stress führt dazu, dass diese Zellen nur schwer in Kultur wachsen und/oder sehr schnell ihre für die Therapie notwendigen Eigenschaften verlieren, wie beispielsweise die Pluripotenz bei iPS. Einmal verloren, sind diese nicht wieder zu gewinnen. Die Zellpopulation ist für die Therapie nicht mehr einsetzbar.

Die erfindungsgemäße Vorrichtung, die die Prozessiereinheit gemäß der dritten Variante aufweist, kann die Kulturbedingungen für solche Zellen selbstständig und lückenlos überwachen und in Echtzeit regulieren. Bisher müssen Mensch oder Roboter die Bedingungen messen und diese anpassen. Dazu wird die Platte mit den Zellen aus eben diesen Bedingungen entnommen, der Deckel geöffnet und die Messungen bzw. Anpassungen durchgeführt. Allein dieser Vorgang kann schon zu viel Stress für die Zellen bedeuten. Bisher ist es daher nur mit sehr hohem Aufwand möglich, solche Zellen zu kultivieren. Dies macht die Therapien sehr teuer.

Dieses Problem kann mit einer Vorrichtung gelöst werden, die die Prozessiereinheit gemäß der dritten Variante aufweist. Die Zellen können stressfrei und unter konstant kontrollierten Bedingungen wachsen. Die Sensoren können beispielsweise durch ein Fluoreszenz-Signal ausgelesen werden, welches von der weiteren Prozessiereinheit, insbesondere der weiteren optischen Erfassungseinrichtung, erfasst wird. Somit kann die Analyse komplett innerhalb der Vorrichtung durchgeführt werden und ein Öffnen der Vorrichtung und/oder ein Transfer der Probe zu einem externen Analysegerät ist nicht erforderlich.

Bei einer besonderen Ausführung kann vor einer Prozessierung der flüssigen Probe die elektrische Steuer- oder Regeleinheit programmiert und/oder ein Prozessierungsprogramm ausgewählt und gestartet werden. So kann die elektrische Steuer- oder Regeleinheit derart programmiert werden, dass sie den Prozessierungsschritt gemäß einer vorgegebenen Steuersequenz steuert.

Alternativ oder zusätzlich kann das Prozessierungsprogramm eine Steuersequenz enthalten, gemäß der der Prozessierungsschritt gesteuert wird.

Nachdem die Prozessierung der flüssigen Probe beendet ist, kann die Prozessiereinheit durch eine andere Prozessiereinheit zum Durchführen eines anderen Prozessierungsschritts ausgetauscht werden. Dies ist insbesondere dann vorteilhaft, wenn mittels einer einzigen Prozessiereinheit nicht alle notwendigen Prozessierungsschritte durchgeführt werden können. Da die Prozessiereinheit mit den anderen Bauteilen wieder lösbar verbunden ist, kann der Austausch der Prozessiereinheit auf einfache Weise erfolgen.

Insbesondere kann die andere Prozessiereinheit mit derselben Betätigungseinrichtung wie die Prozessiereinheit verbunden werden. Dies bedeutet, dass dieselbe Betätigungseinrichtung unterschiedliche Prozessierungsschritte von unterschiedlichen Prozessiereinheiten steuern kann.

Darüber hinaus kann nach einer Beendigung der Prozessierung der flüssigen Probe, das Gehäuse und die Betätigungseinrichtung und die Prozessiereinheit von dem Träger getrennt werden. Der Träger kann zu einer Prozessierungsstation transportiert werden, wobei die flüssige Probe in der Prozessierungsstation prozessiert werden kann. Dies ist beispielsweise dann notwendig, wenn komplexe Prozessierungsschritte zum Einsatz kommen, welche mit der Vorrichtung nicht realisierbar sind. Die komplexen Prozessierungsschritte können weiterhin auf konventionelle Art und Weise auf etablierten Laborgeräten durchgeführt werden.

Die von dem Träger getrennte Betätigungseinrichtung kann zu einer Aufladestation transportiert werden, bei der ein elektrischer Speicher der Betätigungseinrichtung mit elektrischer Energie aufgeladen wird. Darüber hinaus kann die von dem Träger getrennte Betätigungseinrichtung zu einer Füllstation transportiert werden, bei der ein Prozessierungsmedium in den Tank der Betätigungseinrichtung zugeführt wird. Die Prozessiereinheit kann zu einer Waschstation transportiert werden, bei der die Prozessiereinheit gewaschen wird. Im Ergebnis kann die Betätigungseinrichtung und/oder die Prozessiereinheit mehrmals verwendet werden, was aus Kostengesichtspunkten vorteilhaft ist.

Bei der besonderen Ausführung kann der Zusammenbau oder Abbau der Vorrichtung manuell oder automatisch durchgeführt werden. Insbesondere kann der Zusammenbau oder Abbau der Vorrichtung jeweils durch eine einzige Handhabungsvorrichtung durchgeführt werden. Dies bietet den Vorteil, dass die bereits vorhandenen Laborgeräte eingesetzt werden können, sodass kein zusätzlicher Investitionsaufwand für neue Laborgeräte anfällt.

Bei einem Zusammenbau der Vorrichtung kann mittels einer Handhabungsvorrichtung, wie beispielsweise einem Roboter, aus einer Vielzahl von unterschiedlich ausgeführten Prozessiereinheiten eine Prozessiereinheit ausgewählt und zu einer Zusammenbaustation transportiert werden, in der die Prozessiereinheit auf den Träger aufgesetzt wird. So können Prozessiereinheiten gemäß der ersten bis dritten Variante zur Verfügung stehen. Darüber hinaus kann die Handhabungsvorrichtung die weitere Prozessiereinheit zu der Zusammenbaustation transportieren. Dies kann vorzugsweise vor einem Anordnen des Trägers in der Zusammenbaustation erfolgen, da der Träger auf die weitere Prozessiereinheit aufgesetzt ist.

Anschließend kann eine zur Prozessiereinheit zugehörige Betätigungseinrichtung aus einer Vielzahl von unterschiedliche ausgeführten Betätigungseinrichtungen ausgewählt und zu der Zusammenbaustation transportiert werden, in der sie auf die Prozessiereinheit aufgesetzt wird. Im Ergebnis können aufgrund des modularen Aufbaus der Vorrichtung die für die Prozessierung der flüssigen Probe notwendigen Bauteile je nach Bedarf ausgewählt und miteinander verbunden werden.

Insbesondere kann eine Steuereinheit der Handhabungsvorrichtung je nach Applikation entscheiden, welche Prozessiereinheiten und Betätigungseinrichtungen ausgewählt und zusammengebaut werden sollen. Ein Beispiel wäre, unterschiedliche Kultur-Bedingungen durch unterschiedliche Vorrichtungen zu realisieren. Wiederum könnte eine andere Vorrichtung eine Lyse der Zellen mit anschließender Amplifikation des Genoms durchführen. Eine weitere Vorrichtung könnte einen Mediumwechsel bei Zellkolonien durchführen. Eine alternative Vorrichtung könnte den Sauerstoffgehalt, den pH-Wert und die Populationsdichte von Zellpopulationen in dem Träger bestimmen. Eine alternative Vorrichtung könnte Verdünnungsreihen für Proteinfaltung oder Synthese zusammenstellen und die Ergebnisse direkt mit der weiteren optischen Erfassungseinrichtung der weiteren Prozessiereinheit auslesen.

Nach einem Zusammenbau der Vorrichtung in der Zusammenbaustation kann die zusammengebaute Vorrichtung zu einer Ablagestation transportiert werden. Die Vorrichtung kann so lange in der Ablagestation verbleiben, bis die Prozessierung beendet ist. Darüber hinaus kann in der Ablagestation die Vorrichtung, insbesondere der elektrische Speicher, mit elektrischer Energie versorgt werden. Natürlich ist eine Prozessierung der flüssigen Probe aufgrund des elektrischen Speichers auch dann möglich, wenn die Vorrichtung nicht mit elektrischer Energie versorgt wird.

Bei einer besonderen Ausführung kann der Transport der Prozessiereinheit und der Betätigungseinrichtung zu den einzelnen Stationen durch dieselbe Transportvorrichtung der Handhabungsvorrichtung durchgeführt werden. Dies bedeutet, dass nur eine einzige Transportvorrichtung zum Zusammenbau der Vorrichtung und zum Transport der Vorrichtung, insbesondere der Betätigungseinrichtung, der Prozessiereinheit sowie des Trägers benötigt wird. Die Transportvorrichtung kann durch eine externe Steuervorrichtung gesteuert werden.

In den Figuren ist der Erfindungsgegenstand schematisch dargestellt, wobei gleiche oder gleichwirkende Bauteile zumeist mit den gleichen Bezugszeichen versehen sind. Dabei zeigt:
Fig. 1 eine Explosionsdarstellung der erfindungsgemäßen Vorrichtung gemäß einem ersten Ausführungsbeispiel,
Fig. 2 eine perspektivische Darstellung der Betätigungseinrichtung,
Fig. 3 eine Draufsicht auf die in Figur 2 gezeigte Betätigungseinrichtung,
Fig. 4 eine Seitenschnittansicht einer Prozessiereinheit gemäß einem ersten Ausführungsbeispiel und einem Träger,
Fig. 5 eine perspektivische Darstellung einer Prozessiereinheit gemäß einem zweiten Ausführungsbeispiel und einem Träger,
Fig. 6 eine Seitenschnittansicht der in Figur 5 gezeigten Prozessiereinheit,
Fig. 7 eine Explosionsdarstellung der Betätigungseinrichtung und der Prozessiereinheit gemäß einem dritten Ausführungsbeispiel
Fig. 8 eine vergrößerte Darstellung eines Teils der in Fig. 7 gezeigten Betätigungseinrichtung und der Prozessiereinheit
Fig. 9 eine Seitenschnittansicht der Prozessiereinheit gemäß einem vierten Ausführungsbeispiel und einem Träger,
Fig. 10 eine Draufsicht auf eine Analyseeinheit,
Fig. 11 eine Draufsicht auf eine Prozessiereinheit gemäß einem fünften Ausführungsbeispiel,
Fig. 12 eine Seitenschnittansicht einer Prozessiereinheit gemäß einem sechsten Ausführungsbeispiel,
Fig. 13 eine Seitenschnittansicht einer Prozessiereinheit gemäß einem siebten Ausführungsbeispiel,
Fig. 14 eine Seitenschnittansicht einer Prozessiereinheit gemäß einem achten Ausführungsbeispiel mit einem Träger und einem Aufnahmeelement,
Fig. 15 eine Handhabungsvorrichtung für die erfindungsgemäße Vorrichtung.

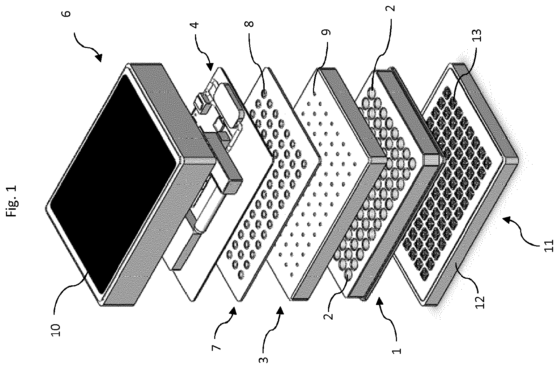

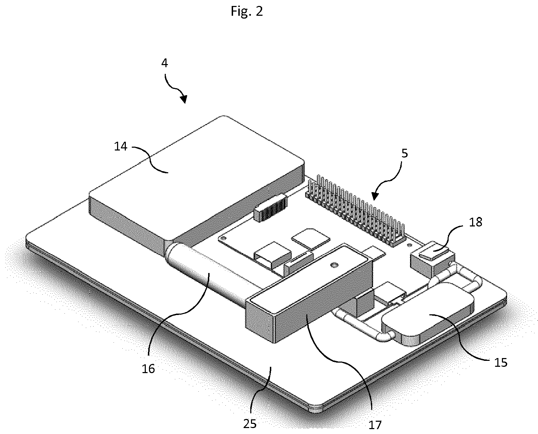

Die in Figur 1 gezeigte Vorrichtung zur Prozessierung einer flüssigen Probe weist einen Träger 1 auf, der eine Vielzahl von Behältnissen 2 aufweist. In den Behältnissen 2 ist jeweils eine flüssige Probe angeordnet. Darüber hinaus weist die Vorrichtung eine Prozessiereinheit 3 zum Durchführen wenigstens eines Prozessierungsschritts und eine Betätigungseinrichtung 4 auf. Die Betätigungseinrichtung 4 weist eine in Figur 2 dargestellte elektrische Steuer- oder Regeleinheit 5 auf, die zum Steuern oder Regeln des mittels der Prozessiereinheit 3 durchgeführten Prozessierungsschritts oder Prozessierungsschritte dient. Die elektrische Steuer- oder Regeleinheit 5 kann beispielsweise ein Microcontroller, ein Prozessor oder ähnliches sein. Die Betätigungseinrichtung 4 ist auf die Prozessiereinheit 3 aufgesetzt. Darüber hinaus weist die Vorrichtung ein Gehäuse 6 und ein Zwischenelement 7 auf.

Die Prozessiereinheit 3 ist auf den Träger 1, insbesondere unmittelbar, aufgesetzt und schließt diesen, insbesondere die Behältnisse, im aufgesetzten Zustand hermetisch ab. Dabei ist die Prozessiereinheit 3 wieder lösbar mit dem Träger 1 verbunden. Die Betätigungseinrichtung 4 ist mittels des Zwischenelements 7 mit der Prozessiereinheit 3 fluidisch verbunden. Dazu weist das Zwischenelement 7 eine Vielzahl von Schnittstellen in Form von Durchbrüchen 8 auf. Die Anzahl der Durchbrüche 8 in dem Zwischenelement 7 entspricht der Anzahl von Öffnungen 9 in der Prozessiereinheit 3.

Das Gehäuse 6 wird auf die Betätigungseinrichtung 4, das Zwischenelement 7, die Prozessiereinheit 3 und den Träger 1 gesetzt und ist mit dem Träger 1 wieder lösbar verbunden. In einem zusammengebauten Zustand der Vorrichtung sind die Prozessiereinheit 3, das Zwischenelement 7 und die Betätigungseinrichtung 4 und ein Teil des Trägers 1 innerhalb eines Hohlraums des Gehäuses 6 angeordnet. Das Gehäuse weist einen Touchdisplay 10 auf, mittels dem eine Programmierung der elektrischen Steuer-oder Regeleinheit 5 und/oder das Auswählen eines Programmierungsprogramms erfolgen kann.

Darüber hinaus weist die Vorrichtung eine weitere Prozessiereinheit 11 und eine in Figur 1 nicht dargestellte weitere Betätigungseinrichtung auf. Die weitere Prozessiereinheit 11 und die weitere Betätigungseinrichtung sind in einem weiteren Hohlraum eines weiteren Gehäuses 12 angeordnet. Die weitere Prozessiereinheit 11 weist eine optische Erfassungseinrichtung mit einer Vielzahl von optischen Abbildungsvorrichtungen 13 auf. Mittels der optischen Erfassungseinrichtung kann eine Eigenschaft der flüssigen Probe erfasst werden.

Zum Erfassen der Eigenschaft der flüssigen Probe kann mittels der optischen Abbildungsvorrichtung 13 eine Abbildung der flüssigen Probe erzeugt werden. Dabei kann jedem Behältnis 2 eine optische Abbildungsvorrichtung 13 zugeordnet sein, so dass die Eigenschaften aller flüssigen Proben, die in dem Träger angeordnet sind, mittels der optischen Erfassungseinrichtung erfasst werden können. Die nicht dargestellte weitere Betätigungseinrichtung kann mit der Betätigungseinrichtung 4, insbesondere der elektrischen Steuer oder Regeleinheit 5, datentechnisch kommunizieren.

Die weitere Prozessiereinheit 11 ist an einem der Prozessiereinheit 3 abgewandten Ende des Trägers 1 angeordnet. Der Träger ist auf der weiteren Prozessiereinheit 11 aufgesetzt. Zudem ist die weitere Prozessiereinheit 11 wieder lösbar mit dem Träger 1 verbunden. Die weitere Betätigungseinrichtung ist wieder lösbar mit der weiteren Prozessiereinheit 11 verbunden. Außerdem ist das weitere Gehäuse 12 mit dem Träger 1 wieder lösbar verbunden.

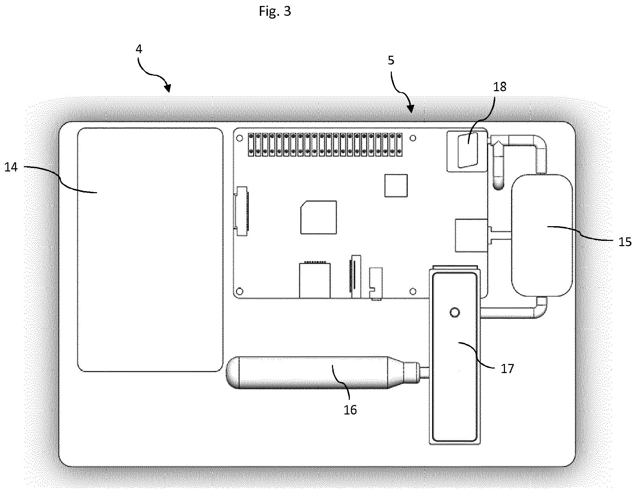

Figur 2 zeigt eine perspektivische Darstellung der Betätigungseinrichtung 4 und Figur 3 zeigt eine Draufsicht auf die Betätigungseinrichtung 4. Die Betätigungseinrichtung 4 weist neben der elektrischen Steuer- oder Regeleinheit 5 einen elektrischen Speicher 14, wie beispielsweise eine wieder aufladbaren Batterie, auf, der die elektrische Steuer-oder Regeleinheit 5 mit elektrischer Energie versorgt. Darüber hinaus weist die Betätigungseinrichtung 4 eine Mikropumpe 15 auf, die mit der Prozessiereinheit 3 fluidisch verbunden ist. Ein Gastank 16, der beispielsweise mit Kohlenstoffdioxid gefüllt ist, ist mittels eines Ventils 17 mit der Mikropumpe 15 und/oder der in Figur 1 dargestellten Prozessiereinheit 3 fluidisch verbunden. Darüber hinaus weist die Betätigungseinrichtung 4 einen Drucksensor 18 und ein nicht dargestelltes Kommunikationsmittel auf. Die einzelnen Bauteile der Betätigungseinrichtung 4 sind auf einer Platte 25 angeordnet, so dass die Betätigungseinrichtung 4 als Modul ausgeführt ist.

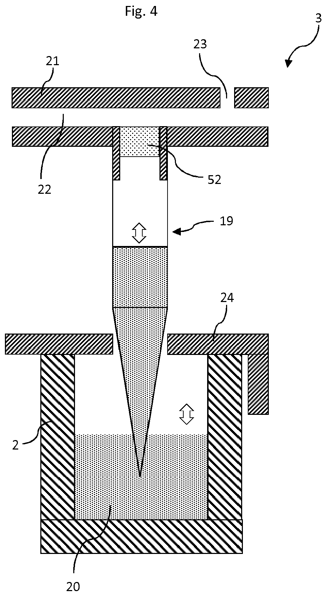

Figur 4 zeigt eine Prozessiereinheit gemäß einem zweiten Ausführungsbeispiel, die nach dem gleichen Prinzip arbeitet, wie die in Figur 1 dargestellte Prozessiereinheit 3. So weist die Prozessiereinheit 3 eine Fluidleitung 19 auf, die in die flüssige Probe 20 eines Behältnisses 2 ragt. Das Behältnis 2 wird mittels eines Deckels 24 der Prozessiereinheit 3 abgedeckt. Die Fluidleitung 19 erstreckt sich durch einen im Deckel 24 vorhandenen Durchbruch hindurch, um in das Behältnis 2 einzudringen.

Die Prozessiereinheit 3 weist außerdem einen Aufsatz 21 auf, von dem sich die Fluidleitung 19 in Richtung zur flüssigen Probe 20 erstreckt. Der Aufsatz 21 weist einen Fluidkanal 22 auf, der fluidisch mit der Fluidleitung 19 verbunden ist. Darüber hinaus ist der Fluidkanal 22 mit einer Auslassöffnung 23 fluidisch verbunden, die mit der Mikropumpe 15 fluidisch verbunden ist. In dem von der flüssigen Probe 20 entfernten Ende der Fluidleitung 19 kann ein Filter 52 angeordnet sein. Der Filter 52 ist flüssigkeitsundurchlässig und gasdurchlässig.

Mittels der Prozessiereinheit 3 kann ein Teil der im Behältnis 2 befindlichen flüssigen Probe 20 in die Fluidleitung 19 eingesaugt und im Anschluss daran in das Behältnis 2 dispensiert werden. Dadurch wird eine Durchmischung der flüssigen Probe 20 in dem Behältnis 2 realisiert. Infolge des Vorgangs des Einsaugens und Absaugens ändert sich der Pegel der flüssigen Probe in dem Behältnis 2 und der Fluidleitung 19 wie durch die Doppelpfeile dargestellt ist. Darüber hinaus kann mittels der Fluidleitung 19 ein Fluid, insbesondere ein Gas oder eine Flüssigkeit, in die flüssige Probe 20 eingebracht werden oder aus dem Behältnis 2 über die Fluidleitung 19 und den Auslass 1 abgeführt werden.

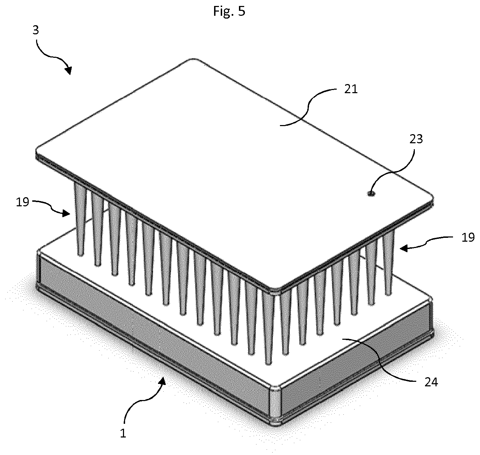

Figur 5 zeigt eine Prozessiereinheit 3 gemäß einem dritten Ausführungsbeispiel, die auf den Träger 1 gesetzt ist. Im Unterschied zu der in Figur 4 dargestellten Ausführung weist die Prozessiereinheit 3 eine Vielzahl von Fluidleitungen 19 auf, die sich von dem Aufsatz 21 in Richtung zu dem Träger 1 erstrecken. Jede der Fluidleitungen 19 dringt dabei in ein im Träger 1 vorhandenes Behältnis 2 und ragt in die jeweilige flüssige Probe. Dabei kann mittels jeder der Fluidleitungen die gleiche Betriebsweise, insbesondere Einsaugen der flüssigen Proben, Dispensieren der flüssigen Probe oder Durchmischen der flüssigen Probe, realisiert werden wie mit der in Figur 4, 5 und 6 dargestellten Fluidleitung 19. Jede der Fluidleitungen 19 kann einen Filter 52 aufweisen.

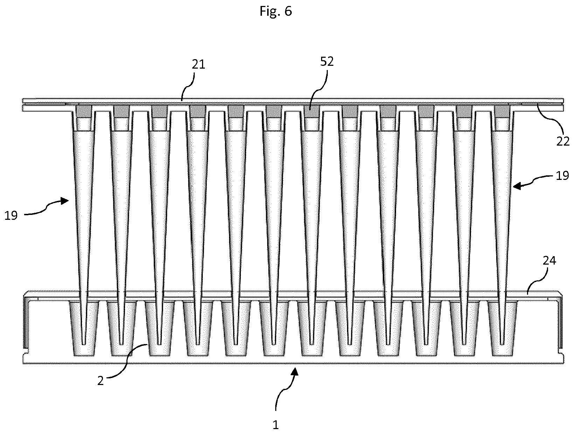

Jede der in Figur 6 dargestellten Fluidleitungen 19 ist mit dem Fluidkanal 22 fluidisch verbunden. Die in Figur 5 dargestellten Fluidleitungen 19, die in Figur 6 nicht dargestellt sind und die in einer anderen Ebene angeordnet sind, die parallel zu der Ebene liegt, die die in Figur 6 gezeigten Fluidleitungen 19 aufweist, können ebenfalls mit dem Fluidkanal 22 fluidisch verbunden sein. Natürlich sind auch Ausführungen denkbar, bei denen nicht alle Fluidleitungen 19 mit dem Fluidkanal 22, sondern mit einem anderen nicht dargestellten Fluidkanal fluidisch verbunden sind. Dabei ist der andere Fluidkanal mit dem Fluidkanal 7 fluidisch nicht verbunden. In diesem Fall weist der Aufsatz 1 noch eine weitere nicht dargestellte Öffnung auf, die mit einer anderen nicht dargestellten Pumpe und/oder einem anderen Tank fluidisch verbunden ist.

Die Fluidleitungen 19 erstrecken sich jeweils unmittelbar vom Aufsatz 21 und sind mit diesem wieder lösbar verbunden. Dabei sind die Fluidleitungen 19 dazu bestimmt und entsprechend ausgebildet, dass sie jeweils in eine in dem Behältnis 2 befindliche flüssige Probe 20 eintauchen. Die flüssige Probe 20 ist in Figur 6 nicht dargestellt.

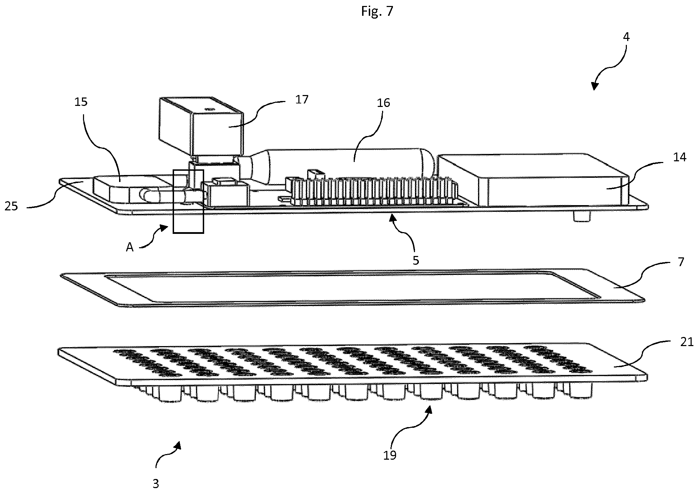

Figur 7 zeigt eine Explosionsdarstellung der Betätigungseinrichtung und der Prozessiereinheit gemäß einem dritten Ausführungsbeispiel. Ein Unterschied zu der in Figur 1 dargestellten Ausführung besteht darin, dass das Zwischenelement 7 nicht als Platte mit Schnittstellen ausgebildet ist, sondern als ein Kleber, mittels dem die Prozessiereinheit 3 mit der Betätigungseinrichtung 4 stoffschlüssig verbunden wird.

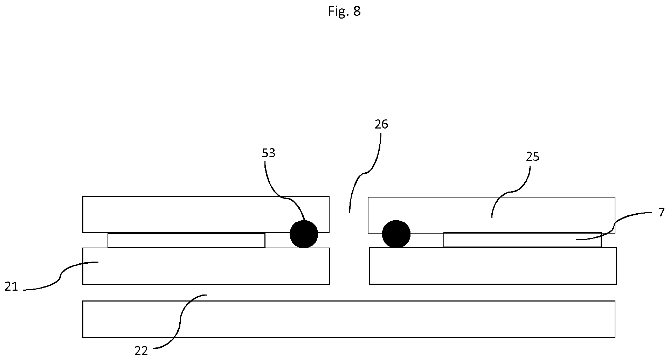

Figur 8 zeigt eine vergrößerte Darstellung des in Figur 7 mit dem Buchstaben A markierten Abschnitts. Aus Figur 8 ist zu entnehmen, dass in der Platte 25 der Betätigungseinrichtung ein Durchlass 26 vorhanden ist, der in fluidischer Verbindung mit dem im Aufsatz 21 vorhandenen Fluidkanal 22 ist. Darüber hinaus ist aus Figur 8 zu entnehmen, dass eine andere Dichtung 53 vorhanden ist, mittels der vermieden wird, dass das durch den Durchlass 26 strömende Fluid mit dem Zwischenelement 7 in Kontakt gelangt.

Mittels der in den Figuren 1, 4, 5, 6 und 7 dargestellten Prozessiereinheiten 3 können die gleichen Betriebsweisen realisiert werden. So kann mittels dieser Prozessiereinheit ein Durchmischen der flüssigen Probe 20 realisiert werden. Darüber hinaus sollen die Prozessiereinheit ermöglichen, dass ein Fluid, insbesondere ein Gas oder eine Flüssigkeit, in ein Behältnis des Trägers 1 zugeführt werden kann. Außerdem soll es mittels der Prozessiereinheit 3 möglich sein, dass ein Teil der in den Behältnissen befindlichen flüssigen Probe in die Fluidleitung 19 eingesaugt wird. Der eingesaugte Teil des Fluids kann wieder in das Behältnis 2 dispensiert oder aus dem Behältnis 2, beispielsweise in ein anderes Behältnis transportiert werden.

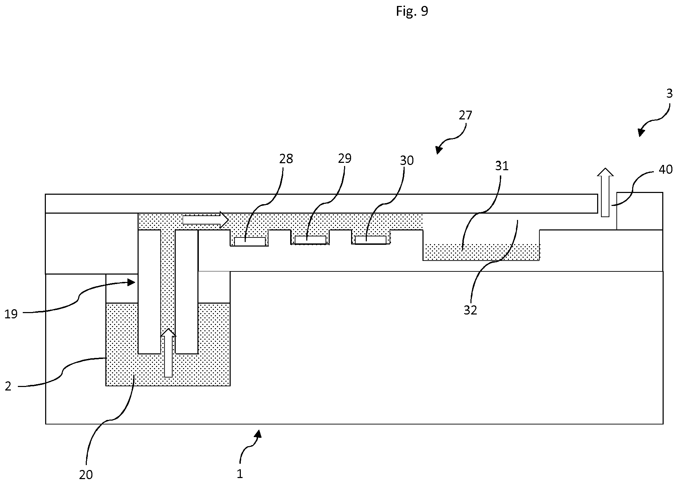

Figur 9 zeigt eine Prozessiereinheit 3 gemäß einem vierten Ausführungsbeispiel. Mittels der Prozessiereinheit 3 soll die in dem Behältnis 2 befindliche flüssige Probe 20 analysiert werden. Die Prozessiereinheit 3 weist eine Analyseeinheit 27 auf, mittels der die flüssige Probe 20 analysiert wird. Die Analyseeinheit 27 weist einen Biosensor 28, einen Temperatursensor 29 und einen Sauerstoffsensor 30 auf. Darüber hinaus weist die Analyseeinheit 27 eine Ausschusskammer 31 auf.

Zum Analysieren der flüssigen Probe 20 wird mittels der Fluidleitung 19 ein Teil der flüssigen Probe 20 in einen Innenraum 32 der Prozessiereinheit 3 eingesaugt. Der in den Innenraum 32 eingeströmte Teil der flüssigen Probe 20 gelangt durch Druck und/oder Kapillarkräfte zu dem Biosensor 28, dem Temperatursensor 29 und dem Sauerstoffsensor 30. Anschließend wird der eingesaugte Teil der flüssigen Probe in die Ausschusskammer 31 geleitet. Das Einströmen des Teils der flüssigen Probe 20 in den Innenraum 32 wird erreicht, indem im Innenraum 32 ein Unterdruck erzeugt wird. Dies kann durch die Mikropumpe 15 erfolgen, weil sie mittels einer weiteren Öffnung 40 mit dem Innenraum 32 in fluidischer Verbindung steht. Die einzelnen Bauteile der Analyseeinheit 27 sind miteinander fluidisch verbunden.

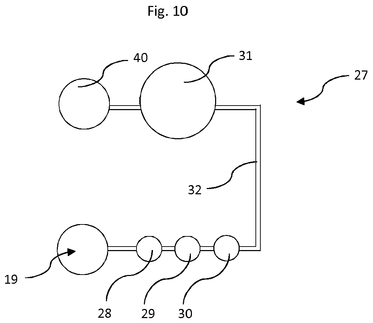

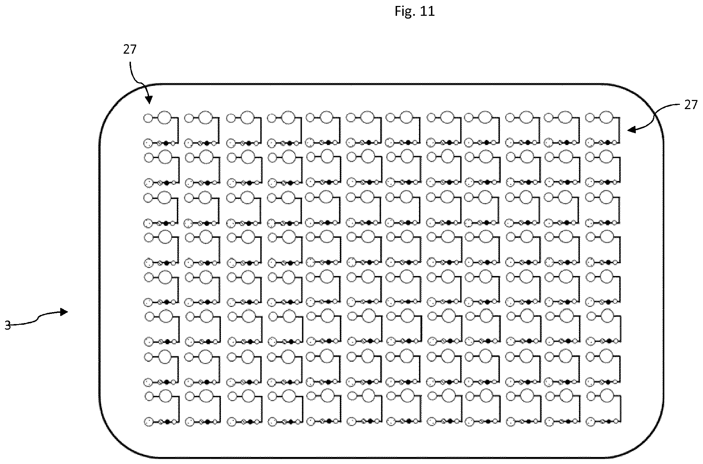

Figur 10 zeigt eine Draufsicht auf eine Analyseeinheit 27, die nach dem gleichen Prinzip wie die die in Figur 9 dargestellte Analyseeinheit 27 arbeitet. Sie unterscheidet sich von der in Figur 9 dargestellten Ausführung in der Anordnung der einzelnen Bestandteile der Analyseeinheit. Figur 11 zeigt eine Draufsicht auf die Prozessiereinheit 3, die eine Vielzahl von in der Figur 10 dargestellten Analyseeinheiten 27 aufweist. Dabei zeigt Figur 11 die Prozessiereinheit 3 ohne Deckel, der die Analyseeinheiten 27 abdeckt. Im Betrieb weist die Prozessiereinheit 3 den Deckel zum Abdecken der Analyseeinheiten 27 auf. Die Analyseeinheiten 27 sind in Reihen und Spalten angeordnet. Die Prozessiereinheit 3 wird auf einen Träger 1 gesetzt, der eine Vielzahl von Behältnissen 2 aufweist. Im Ergebnis kann mittels der Prozessiereinheit 3 eine Analyse jeder in einem Behältnis 2 befindlichen flüssigen Probe erfolgen.

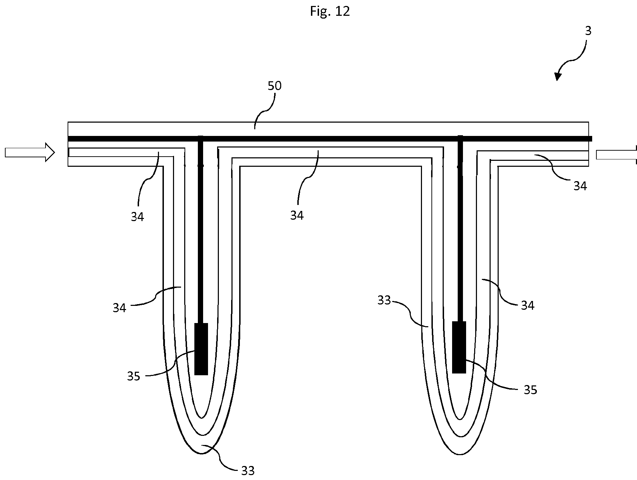

Figur 12 zeigt eine Prozessiereinheit gemäß einem sechsten Ausführungsbeispiel. Die Prozessiereinheit dient zum Erhitzen oder Kühlen einer in Figur 12 nicht dargestellten flüssigen Probe. Die Prozessiereinheit 3 weist ein Abdeckelement 50 von dem sich zwei Stege 33 in die gleiche Richtung erstrecken. Darüber hinaus weist die Prozessiereinheit 3 einen weiteren Fluidkanal 34 auf, in dem ein Heizmittel oder Kühlmittel strömt. Der Fluidkanal 34 erstreckt sich sowohl durch das Abdeckelement 50 als auch durch jeden der beiden Stege 33. Darüber hinaus weist die Prozessiereinheit 3 in jedem der beiden Stege 33 einen weiteren Temperatursensor 35 auf.

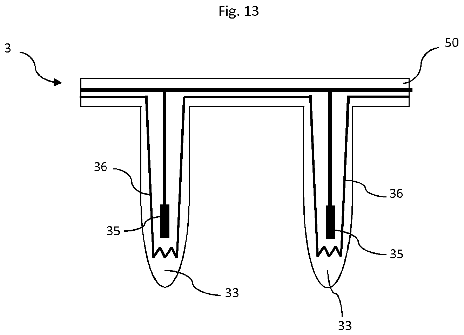

Figur 13 zeigt eine Prozessiereinheit gemäß einem siebten Ausführungsbeispiel, die ebenfalls zum Erhitzen oder Kühlen eine in Figur 13 nicht dargestellten flüssigen Probe dient. Die Prozessiereinheit 3 unterscheidet sich von der in Figur 12 dargestellten Prozessiereinheit 3 darin, dass sie keinen weiteren Fluidkanal 34 aufweist, sondern in jedem der beiden Stege 33 ein Heiz- und/oder Kühlelement 36 vorhanden ist.

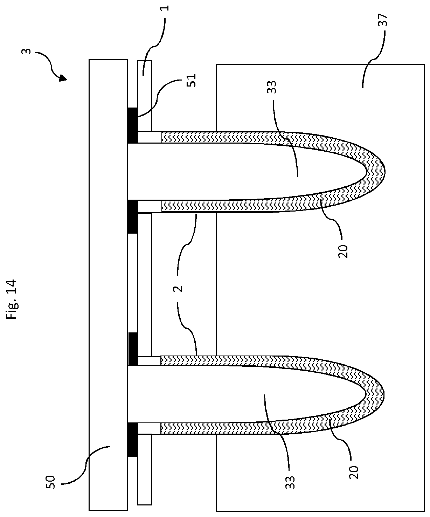

Figur 14 zeigt eine Prozessiereinheit 3, die zum Kühlen oder Heizen der flüssigen Probe 20 dient und die auf einen Träger 1 gesetzt ist. Dabei dringen die Stege 33 in das jeweilige Behältnis 2 des Trägers ein. Die Anzahl der Stege 33 entspricht dabei der Anzahl der im Träger 1 vorhandenen Behältnisse. Die in dem Behältnis 2 befindliche flüssige Probe 20 kann mittels der Stege 33 gekühlt oder aufgeheizt werden. Dabei können die Stege 33 wie in Figur 12 oder 13 dargestellt ist, ausgeführt sein. Mittels des Abdeckelements 50, das eine Dichtung 51 aufweist, wird die flüssige Probe 20 abgedichtet.

Der Träger 1 ist auf ein Aufnahmeelement 37 gesetzt. Insbesondere sind die Behältnisse 2 des Trägers 1 zum Teil in dem Aufnahmeelement 37 angeordnet. Das Aufnahmeelement 37 kann auch eine Kühlfunktion haben, wenn es eine geringere Temperatur aufweist als die flüssige Probe 20, oder es kann zur thermischen Isolation der Probe gegenüber der in der Umgebung herrschenden Temperatur dienen.

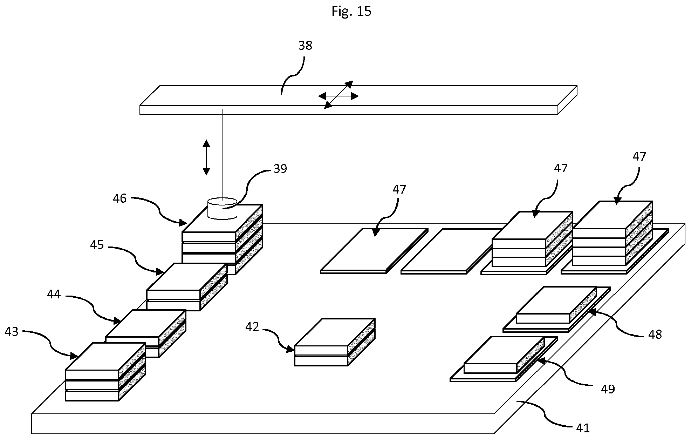

Figur 15 zeigt eine, insbesondere automatisch arbeitende, Handhabungsvorrichtung für die Vorrichtung. Die Handhabungsvorrichtung dient im Wesentlichen zum Zusammenbau, Abbau oder Transport der Vorrichtung. Sie weist eine Transportvorrichtung 38 mit einem Greifer 39 und eine Plattform 41 auf, die mehrere Stationen aufweist. Die Handhabungsvorrichtung wird mittels einer nicht dargestellten Steuereinrichtung gesteuert.

Die Plattform 41 weist eine Zusammenbaustation 42 auf, in der ein Zusammenbau der Vorrichtung erfolgt. Darüber hinaus weist die Plattform mehrere Lagerstationen auf, bei der die unterschiedlichen Bauteile der Vorrichtung gelagert sind. So weist die Plattform eine erste Lagerstation 43, in der Prozessiereinheiten gelagert werden und eine zweite Lagerstation 44 auf, in der weitere Prozessiereinheiten gelagert werden. Natürlich kann die Plattform auch weitere Lagerstationen für Prozessiereinheiten gemäß der oben beschriebenen unterschiedlichen Varianten aufweisen.

Darüber hinaus weist die Plattform eine dritte Lagerstation 45 und eine vierte Lagerstation 46 auf. In der dritten Lagerstation 45 werden Bearbeitungseinrichtungen gemäß einer Variante und in der vierten Lagerstation 46 werden Bearbeitungseinrichtungen gemäß einer anderen Variante gelagert.

Darüber hinaus weist die Plattform 41 mehrere Ablagestationen 47 auf, auf die die fertig zusammengebauten Vorrichtungen abgelegt werden können. Die Vorrichtungen können in der Ablagestation 47 mit elektrischer Energie versorgt werden. Außerdem weist die Plattform eine Ladestation, in der der Tank der Betätigungseinrichtung und/oder der weitere Tank der Prozessiereinheit mit dem Prozessierungsmedium aufgeladen werden können. Darüber hinaus weist die Plattform 41 eine Waschstation 49, bei der bereits benutzte Prozessiereinheiten gewaschen werden können.

Die nicht dargestellte Steuereinrichtung entscheidet autonom abhängig von den flüssigen Proben und des benötigten Prozesses welche der Prozessiereinheiten und der Betätigungseinrichtungen zu der Zusammenbaustation 42 transportiert und dort zusammengebaut werden sollen. Nach dem Zusammenbau der Vorrichtung werden diese in der Ablagestation 47 abgelegt, wo die flüssige Probe prozessiert wird.

Nach Beendigung der Prozessierung können die einzelnen Bestandteile der Vorrichtung von dem Träger entfernt werden. So wird die Betätigungseinrichtung zu der Ladestation 48 transportiert, in der ihr das Prozessierungsmedium zugeführt wird. Die Prozessiereinheit kann zur Reinigung in die Waschstation 49 transportiert werden. Anschließend können die Prozessiereinheit und/oder die Betätigungseinrichtung zu ihrer zugehören Lagerstation 43-46 transportiert und dort abgelegt werden.

### Bezuqszeichenliste:

- 1: Träger
- 2: Behältnis
- 3: Prozessiereinheit
- 4: Betätigungseinrichtung
- 5: elektrische Steuer-oder Regeleinheit
- 6: Gehäuse
- 7: Zwischenelement
- 8: Durchbruch
- 9: Öffnung
- 10: Touchdisplay
- 11: weitere Prozessiereinheit
- 12: weiteres Gehäuse
- 13: optische Abbildungsvorrichtung
- 14: elektrische Speicher
- 15: Mikropumpe
- 16: Gastank
- 17: Ventil
- 18: Drucksensor
- 19: Fluidleitung
- 20: flüssige Probe
- 21: Aufsatz
- 22: Fluidkanal
- 23: Auslassöffnung
- 24: Deckel
- 25: Platte
- 26: Durchlass
- 27: Analyseeinheit
- 28: Biosensor
- 29: Temperatursensor
- 30: Sauerstoffsensor
- 31: Ausschusskammer
- 32: Innenraum
- 33: Steg
- 34: weiterer Fluidkanal
- 35: weiterer Temperatursensor
- 36: Heiz- und/oder Kühlelement
- 37: Aufnahmeelement
- 38: Transportvorrichtung
- 39: Greifer
- 40: weitere Öffnung
- 41: Plattform
- 42: Zusammenbaustation
- 43: erste Lagerstation
- 44: zweite Lagerstation
- 45: dritte Lagerstationen
- 46: vierte Lagerstation
- 47: Ablagestation
- 48: Ladestation
- 49: Waschstation
- 50: Abdeckelement
- 51: Dichtung
- 52: Filter
- 53: andere Dichtung

## Patentansprüche

1. Vorrichtung zur Prozessierung einer flüssigen Probe, mit einem Träger (1), der wenigstens ein Behältnis (2) zum Aufnehmen der flüssigen Probe aufweist, einer Prozessiereinheit (3) zum Durchführen wenigstens eines Prozessierungsschritts und einer Betätigungseinrichtung (4), die eine elektrische Steuer- oder Regeleinheit (5) zum Steuern oder Regeln des mittels der Prozessiereinheit (3) durchgeführten Prozessierungsschritts aufweist, **dadurch gekennzeichnet, dass** die Betätigungseinrichtung (4), insbesondere die Steuer- oder Regeleinheit (5) auf die Prozessiereinheit (3) aufgesetzt ist und dass die Prozessiereinheit (3) auf den Träger (1) aufgesetzt ist.

2. Vorrichtung nach Anspruch 1, **dadurch gekennzeichnet, dass** die Vorrichtung ein Gehäuse (6) aufweist,
a. das mit dem Träger (1) wieder lösbar verbunden ist und den Träger (1) abschließt und/oder dass
b. die Betätigungseinrichtung (4) zwischen dem Gehäuse (6) und dem Träger (1) angeordnet ist und/oder dass
c. das Gehäuse (6) auf die Betätigungseinrichtung (4) aufgesetzt ist und/oder dass
d. die Vorrichtung derart ausgebildet ist, dass die Prozessierung der flüssigen Probe in der Vorrichtung autonom erfolgt und/oder dass
e. das Gehäuse (6) und/oder die Betätigungseinrichtung (4) und/oder die Prozessiereinheit (3) derart ausgebildet sind, dass sie durch dieselbe Handhabungsvorrichtung handhabbar sind wie der Träger (1) und/oder dass
f. die Betätigungseinrichtung (4) mit der Prozessiereinheit (3) wieder lösbar verbunden ist und/oder dass
g. die Prozessiereinheit (3) mit dem Träger (1) wieder lösbar verbunden ist und/oder dass
h. das Gehäuse (6) wieder lösbar oder fest mit der Betätigungseinrichtung (4) verbunden ist.

3. Vorrichtung nach Anspruch 1 oder 2, **dadurch gekennzeichnet, dass**
a. das Gehäuse (6) eine Eingabevorrichtung, insbesondere ein Touchdisplay (10), zum Eingeben von Daten an die elektrische Steuer- oder Regeleinheit (5) der Betätigungseinrichtung (4) aufweist und/oder dass
b. das Gehäuse (6) kastenartig ausgeführt ist, wobei die Betätigungseinrichtung (4), insbesondere die elektrische Steuer- oder Regeleinheit (5), und/oder die Prozessiereinheit (3) und/oder der Träger (1) in dem Hohlraum des Gehäuses (6) angeordnet sind und/oder dass
c. die Betätigungseinrichtung (4) einen, insbesondere wieder aufladbaren, elektrischen Speicher (14) zum Versorgen der elektrischen Steuer- oder Regeleinheit (5) mit elektrischer Energie aufweist und/oder dass
d. die Betätigungseinrichtung (4) ein Kommunikationsmittel zum, insbesondere kabellosen, Versenden und/oder Empfangen von Daten aufweist und/oder dass
e. die Betätigungseinrichtung (4) wenigstens eine Pumpe aufweist, die durch die elektrische Steuer- oder Regeleinheit (5) ansteuerbar ist und/oder dass
f. die Betätigungseinrichtung (4) wenigstens einen Tank zur Lagerung eines Prozessierungsmediums aufweist, das, insbesondere mittels eines Ventils, mit einer Pumpe und/oder der Prozessiereinheit (3) fluidisch verbindbar ist und/oder dass
g. die Betätigungseinrichtung (4) eine Messeinheit, insbesondere einen Drucksensor (18), aufweist, der mit der elektrischen Steuer- oder Regeleinheit (5) elektrisch verbunden ist und/oder mit der Prozessiereinheit (3) fluidisch verbunden ist und/oder dass
h. die Betätigungseinrichtung (4) eine Platte (25) aufweist, auf der die elektrische Steuer-oder Regeleinheit (5) und/oder der elektrische Speicher (14) und/oder das Kommunikationsmittel und/oder die Pumpe und/oder der Tank und/oder die Messeinheit angeordnet sind.

4. Vorrichtung nach einem der Ansprüche 1 bis 3, **dadurch gekennzeichnet, dass**
a. bei einem Aufsetzen der Betätigungseinrichtung (4) auf die Prozessiereinheit (3) oder bei einem Abnehmen der Betätigungseinrichtung (4) von der Prozessiereinheit (3) das in der Prozessiereinheit (3) befindliche Prozessierungsmedium und/oder der in der Prozessiereinheit (3) befindliche Teil der flüssigen Probe nicht in Kontakt mit der Betätigungseinrichtung (4) gelangen und/oder dass
b. die Betätigungseinrichtung (4) schlauchlos oder kabellos mit der Prozessiereinheit (3) verbunden ist und/oder dass
c. ein Zwischenelement (7) vorhanden ist, das zwischen der Betätigungseinrichtung (4) und der Prozessiereinheit (3) angeordnet ist und/oder dass
d. ein Zwischenelement (7) vorhanden ist, das mittels dem die Betätigungseinrichtung (4) mit der Prozessiereinheit (3) verbunden ist und/oder dass
e. ein Zwischenelement (7) vorhanden ist, das zwischen der Betätigungseinrichtung (4) und der Prozessiereinheit (3) angeordnet ist und/oder mittels dem die Betätigungseinrichtung (4) mit der Prozessiereinheit (3) verbunden ist, wobei die Betätigungseinrichtung (4) mittels des Zwischenelements (3) mit der Prozessiereinheit (3) stoffschlüssig verbunden ist.

5. Vorrichtung nach einem der Ansprüche 1 bis 4, **dadurch gekennzeichnet, dass**
a. die Prozessiereinheit (3) wenigstens einen weiteren Tank zur Lagerung eines weiteren Prozessierungsmediums aufweist und/oder dass
b. die Prozessiereinheit (3) wenigstens eine Fluidleitung (19) aufweist, die sich von einem Aufsatz (21) erstreckt und in die flüssige Probe ragt und/oder dass
c. die Prozessiereinheit (3) wenigstens eine Fluidleitung (19) aufweist, die sich von einem Aufsatz (21) erstreckt und in die flüssige Probe ragt, wobei wenigstens eine Fluidleitung (19) mit einem Fluidkanal (22), der im Aufsatz (21) angeordnet ist, fluidisch verbunden ist und/oder dass
d. die Prozessiereinheit (3) wenigstens eine Fluidleitung (19) aufweist, die sich von einem Aufsatz (21) erstreckt und in die flüssige Probe ragt, wobei wenigstens eine andere Fluidleitung mit einem anderen Fluidkanal, der im Aufsatz (21) angeordnet ist, fluidisch verbunden ist.

6. Vorrichtung nach Anspruch 5c oder 5d, **dadurch gekennzeichnet, dass**
a. der Fluidkanal (22) mit der Pumpe fluidisch verbunden ist und/oder dass
b. der Fluidkanal (22) mit dem Tank und/oder dem weiteren Tank fluidisch verbunden ist und/oder dass
c. der andere Fluidkanal mit einer andere Pumpe fluidisch verbunden ist und/oder dass
d. der andere Fluidkanal mit dem Tank und/oder dem weiteren Tank fluidisch verbunden ist.

7. Vorrichtung nach einem der Ansprüche 1 bis 6, **dadurch gekennzeichnet, dass**
a. die Prozessiereinheit (3) wenigstens ein weiteres Ventils aufweist, das mit der elektrischen Steuer- oder Regeleinheit (5) verbunden ist und/oder dass
b. die Prozessiereinheit (3) ein Heizelement und/oder ein Kühlelement (36) aufweist und/oder dass
c. die Prozessiereinheit (3) einen weiteren Fluidkanal (34) aufweist, in dem ein Heizmittel oder ein Kühlmittel strömbar ist und/oder dass
d. das Behältnis (2) des Trägers (1) in einer Aufnahme eines Aufnahmeelements (37) angeordnet ist und/oder dass
e. die Prozessiereinheit (3) wenigstens eine Analyseeinheit (27) zum Analysieren der flüssigen Probe aufweist und/oder dass
f. die Analyseeinheit (27) wenigstens einen Sensor und eine Ausschusskammer (31) zur Aufnahme des in die Analyseeinheit (27) eingesaugten Teils der flüssigen Probe aufweist, wobei die Ausschusskammer dem Sensor strömungstechnisch nachgeschaltet ist und/oder dass
g. die Prozessiereinheit (3) wenigstens eine optische Erfassungseinrichtung zum Erfassen einer Eigenschaft der flüssigen Probe und/oder des Sensors aufweist und/oder dass
h. die Prozessiereinheit (3) als Einwegbauteil ausgeführt ist.

8. Vorrichtung nach einem der Ansprüche 1 bis 7, **gekennzeichnet durch** eine weitere Prozessiereinheit (11) zum Durchführen wenigstens eines weiteren Prozessierungsschritts und eine weitere Betätigungseinrichtung, die an der von der Prozessiereinheit (3) abgewandten Seite des Trägers (1) angeordnet sind.

9. Vorrichtung nach Anspruch 8, **dadurch gekennzeichnet, dass**
a. die weitere Prozessiereinheit (11) zwischen dem Träger (1) und der weiteren Betätigungseinrichtung angeordnet ist und/oder dass
b. die weitere Prozessiereinheit (11) wenigstens eine weitere optische Erfassungseinrichtung zum Erfassen einer Eigenschaft der flüssigen Probe und/oder des Sensors aufweist und/oder dass
c. der Träger (1) auf die weiter Prozessiereinheit (11) aufgesetzt ist und/oder dass
d. die weitere Prozessiereinheit (11) und/oder die Betätigungseinrichtung (4) in einem weiteren Hohlraum eines weiteren Gehäuses angeordnet sind, wobei das weitere Gehäuse mit dem Träger (1) wieder lösbar verbindbar ist und/oder dass
e. die weitere Betätigungseinrichtung und die Betätigungseinrichtung (4) datentechnisch kommunizieren.

10. Verfahren zur Prozessierung einer flüssigen Probe, **dadurch gekennzeichnet, dass**
a. wenigstens ein Behältnis (2) eines Trägers (1) mit wenigstens einer flüssigen Probe befüllt wird und dass
b. eine Prozessiereinheit (3) auf den Träger (1) aufgesetzt wird und dass
c. eine Betätigungseinrichtung (4) auf die Prozessiereinheit (3) aufgesetzt wird, wobei die Betätigungseinrichtung (4) eine elektrische Steuer- oder Regeleinheit (5) aufweist und dass
d. durch die elektrische Steuer- oder Regeleinheit (5) ein durch die Prozessiereinheit (3) durchgeführter Prozessierungsschritt gesteuert oder geregelt wird.

11. Verfahren nach Anspruch 10, **dadurch gekennzeichnet, dass**
a. ein Gehäuse, das den Träger (1) und die Betätigungseinrichtung (4) und die Prozessiereinheit (3) abschließt mit dem Träger (1) wieder lösbar verbunden wird und/oder dass
b. vor einer Prozessierung der flüssigen Probe die elektrische Steuer- oder Regeleinheit (5) programmiert wird und/oder ein Prozessierungsprogramm ausgewählt und gestartet wird und/oder dass
c. die Steuerung des Prozessierungsschritts gemäß einer vorgegebenen Steuersequenz durchgeführt wird und/oder dass
d. nachdem die Prozessierung der flüssigen Probe beendet ist, die Prozessiereinheit (3) durch eine andere Prozessiereinheit (11) zum Durchführen eines anderen Prozessierungsschritts ausgetauscht wird, insbesondere und die andere Prozessiereinheit (11) mit derselben Betätigungseinrichtung (4) wie die Prozessiereinheit verbunden wird, oder nachdem die Prozessierung der flüssigen Probe beendet ist, das Gehäuse (6) und die Betätigungseinrichtung (4) und die Prozessiereinheit (3) von dem Träger (1) getrennt werden und der Träger (1) zu einer Prozessierungsstation transportiert wird und die flüssige Probe in der Prozessierungsstation prozessiert wird und/oder dass
e. die Betätigungseinrichtung (4) zu einer Füllstation transportiert wird, bei der ein Prozessierungsmedium in einen Tank der Betätigungseinrichtung (4) zugeführt wird und/oder dass
f. die Prozessiereinheit (3) zu einer Waschstation (49) transportiert wird, bei der die Prozessiereinheit (3) gewaschen wird und/oder dass
g. ein Zusammenbau oder Abbau der Vorrichtung manuell oder automatisch durchgeführt wird und/oder dass
h. ein Zusammenbau oder Abbau der Vorrichtung jeweils durch eine einzige Handhabungsvorrichtung durchgeführt wird und/oder dass
i. bei einem Zusammenbau der Vorrichtung aus einer Vielzahl von unterschiedlich ausgeführten Prozessiereinheiten eine Prozessiereinheit (3) ausgewählt und zu einer Zusammenbaustation transportiert wird, in der die Prozessiereinheit auf den Träger aufgesetzt wird und anschließend eine zur Prozessiereinheit (3) zugehörige Betätigungseinrichtung (4) aus einer Vielzahl von unterschiedliche ausgeführten Betätigungseinrichtungen ausgewählt und zu der Zusammenbaustation transportiert wird, in der sie auf die Prozessiereinheit (3) aufgesetzt wird, insbesondere und dass nach einem Zusammenbau der Vorrichtung in der Zusammenbaustation die zusammengebaute Vorrichtung zu einer Ablagestation (47) transportiert wird und/oder dass der Transport der Prozessiereinheit (3) und der Betätigungseinrichtung (4) zu den einzelnen Stationen durch dieselbe Transportvorrichtung (38) durchgeführt wird.

12. Verfahren nach Anspruch 10 oder 11, **dadurch gekennzeichnet, dass**
a. mittels der Prozesseinheit (3) wahlweise ein Durchmischen der in einem Behältnis (2) des Trägers (1) befindlichen flüssigen Probe durchgeführt wird oder ein Einsaugen eines Teils der flüssigen Probe oder ein Dispensieren eines Fluids in die flüssige Probe, insbesondere und dass das Durchmischen der flüssigen Probe dadurch durchgeführt wird, dass abwechselnd und/oder mehrmals hintereinander ein Teil der in dem Behältnis (2) befindlichen flüssigen Probe in eine Fluidleitung der Prozessiereinheit (3) eingesaugt und der in die Fluidleitung (19) eingesaugt Teil der flüssigen Probe anschließend unmittelbar in die flüssige Probe dispensiert wird und/oder dass an einem Auslass der Fluidleitung (19) eine Gasblase erzeugt wird und abwechselnd und/oder mehrmals hintereinander ein Gasblasendurchmesser vergrößert und verkleinert wird, und/oder dass
b. mittels der Prozessiereinheit (3) wenigstens ein Gemisch erzeugt wird, das wenigstens zwei Prozessierungsmedien aufweist, wobei mittels der Prozessiereinheit (3) ein Prozessierungsmedium mit einer vorgegebenen Menge in das Behältnis (2) zugeführt und anschließend mittels der Prozessiereinheit (3) ein anderes Prozessierungsmedium mit einer vorgegebenen Menge in dasselbe Behältnis (2) zugeführt wird, insbesondere und dass anschließend mittels der Prozessiereinheit (3) das Prozessierungsmedium und das andere Prozessierungsmedium in ein anderes Behältnis zugeführt werden, wobei das in dem anderen Behältnis erzeugte andere Gemisch eine andere Menge an dem Prozessierungsmedium und/oder ein andere Menge an dem anderen Prozessierungsmedium aufweist als das in dem Behältnis befindliche Gemisch, und/oder dass
c. mittels der Prozessiereinheit (3) eine flüssige Probe aus einem Behältnis (2) in ein anderes Behältnis, insbesondere unter einer konstanten Flussrate, transferiert wird, insbesondere und dass das Transferieren der flüssigen Probe zirkulierend durch mehrere Behältnisse (2) durchgeführt wird oder dass das Transferieren der flüssigen Probe beendet wird, nachdem die flüssige Probe in ein vorgegebenes Behältnis (2) dispensiert wird.

13. Verfahren nach einem der Ansprüche 10 bis 12, **dadurch gekennzeichnet, dass**
a. das Behältnis (2), insbesondere von der weiteren optischen Erfassungseinrichtung einer weiteren Prozessiereinheit (11), überwacht wird, wobei bei der Überwachung eine Eigenschaft der flüssigen Probe optisch erfasst wird und die elektrische Steuer- oder Regeleinheit (5) den durch die Prozessiereinheit (3) durchgeführten Prozessierungsschritt abhängig von der erfassten Eigenschaft regelt, insbesondere und dass das Mischungsverhältnis in einem Behältnis (2) überwacht wird und/oder dass überwacht wird, ob die flüssige Probe mit einem Fremdkörper befallen ist, vorzugsweise und dass bei einem Befall der flüssigen Probe mit einem Fremdkörper mittels der Prozessiereinheit (3) ein Prozessierungsmedium, insbesondere ein Gegenmittel, in die befallene flüssige Probe eingebracht wird, und/oder dass
b. mittels der Prozessiereinheit (3) die flüssige Probe aufgeheizt oder gekühlt wird, insbesondere und dass ein Heizmittel oder ein Kühlmittel (36) durch die Prozessiereinheit (3) strömt.

14. Verfahren nach einem der Ansprüche 10 bis 13, **dadurch gekennzeichnet, dass** die Prozessiereinheit (3) die flüssige Probe analysiert, insbesondere und dass zum Analysieren der flüssigen Probe sie zu wenigstens einem in der Prozessiereinheit (3) angeordneten Sensor geleitet wird, vorzugsweise und dass der Sensor durch eine optische Erfassungseinrichtung und/oder eine weitere optische Erfassungseinrichtung ausgelesen wird und/oder dass die in die Prozessiereinheit (3) eingesaugte flüssige Probe nach dem Analysieren in einen in der Prozessiereinheit (3) angeordnete Ausschusskammer geleitet wird.

## Claims

1. A device for processing a liquid sample, comprising a carrier (1) which has at least one container (2) for receiving the liquid sample, a processing unit (3) for carrying out at least one processing step, and an actuating apparatus (4) which has an electrical open-loop or closed-loop control unit (5) for controlling or regulating the processing step carried out by means of the processing unit (3), **characterised in that** the actuating apparatus (4), in particular the open-loop or closed-loop control unit (5), is mounted on the processing unit (3), and that the processing unit (3) is mounted on the carrier (1).

2. The device according to claim 1, **characterised in that** the device comprises a housing (6)
a. which is releasably connected to the carrier (1) and closes off the carrier (1) and/or **in that**
b. the actuating apparatus (4) is arranged between the housing (6) and the carrier (1), and/or that
c. the housing (6) is mounted on the actuating apparatus (4), and/or that
d. the device is designed in such a way that the processing of the liquid sample in the device takes place autonomously, and/or that
e. the housing (6) and/orthe actuating apparatus (4) and/orthe processing unit (3) are designed in such a way that they can be handled by the same handling device as the carrier (1), and/or that
f. the actuating apparatus (4) is releasably connected to the processing unit (3), and/or that
g. the processing unit (3) is releasably connected to the carrier (1), and/or that
h. the housing (6) is releasably or firmly connected to the actuating apparatus (4).

3. The device according to claim 1 or 2, **characterised in that**
a. the housing (6) comprises an input device, in particular a touch display (10), for inputting data into the electrical open-loop or closed-loop control unit (5) of the actuating apparatus (4), and/or that
b. the housing (6) is configured like a box, wherein the actuating apparatus (4), in particular the electrical open-loop or closed-loop control unit (5), and/or the processing unit (3) and/or the carrier (1) are arranged in the cavity of the housing (6), and/or that
c. the actuating apparatus (4) comprises an in particular rechargeable, electrical store (14) for supplying the electrical open-loop or closed-loop control unit (5) with electrical energy, and/or that
d. the actuating apparatus (4) comprises a communication means for in particular wirelessly sending and/or receiving data, and/or that
e. the actuating apparatus (4) comprises at least one pump which can be controlled by the electrical open-loop or closed-loop control unit (5), and/or that
f. the actuating apparatus (4) comprises at least one tank for storing a processing medium which can be fluidically connected to a pump and/or the processing unit (3), in particular by means of a valve, and/or that
g. the actuating apparatus (4) comprises a measuring unit, in particular a pressure sensor (18), which is electrically connected to the electrical open-loop or closed-loop control unit (5) and/or is fluidically connected to the processing unit (3), and/or that
h. the actuating apparatus (4) comprises a plate (25) on which the electrical open-loop or closed-loop control unit (5) and/or the electrical store (14) and/or the communication means and/or the pump and/or the tank and/or the measuring unit are arranged.

4. The device according to any one of claims 1 to 3, **characterised in that**
a. when the actuating apparatus (4) is mounted on the processing unit (3) or when the actuating apparatus (4) is removed from the processing unit (3), the processing medium located in the processing unit (3) and/or the portion of the liquid sample located in the processing unit (3) do/does not come into contact with the actuating apparatus (4), and/or that
b. the actuating apparatus (4) is tubelessly or wirelessly connected to the processing unit (3), and/or that
c. an intermediate element (7) is present, which is arranged between the actuating apparatus (4) and the processing unit (3), and/or that
d. an intermediate element (7) is present, by means of which the actuating apparatus (4) is connected to the processing unit (3), and/or that
e. an intermediate element (7) is present, which is arranged between the actuating apparatus (4) and the processing unit (3) and/or by means of which the actuating apparatus (4) is connected to the processing unit (3), wherein the actuating apparatus (4) is integrally connected to the processing unit (3) by means of the intermediate element (3).

5. The device according to any one of claims 1 to 4, **characterised in that**
a. the processing unit (3) comprises at least one further tank for storing a further processing medium, and/or that
b. the processing unit (3) comprises at least one fluid line (19) which extends from an attachment (21) and protrudes into the liquid sample, and/or that
c. the processing unit (3) comprises at least one fluid line (19) which extends from an attachment (21) and protrudes into the liquid sample, wherein at least one fluid line (19) is fluidically connected to a fluid channel (22) arranged in the attachment (21), and/or that
d. the processing unit (3) comprises at least one fluid line (19) which extends from an attachment (21) and protrudes into the liquid sample, wherein at least one other fluid line is fluidically connected to another fluid channel arranged in the attachment (21).

6. The device according to claim 5c or 5d, **characterised in that**
a. the fluid channel (22) is fluidically connected to the pump, and/or that
b. the fluid channel (22) is fluidically connected to the tank and/or the further tank, and/or that
c. the other fluid channel is fluidically connected to another pump, and/or that
d. the otherfluid channel is fluidically connected to the tank and/orthe further tank.

7. The device according to any one of claims 1 to 6, **characterised in that**
a. the processing unit (3) comprises at least one further valve which is connected to the electrical open-loop or closed-loop control unit (5), and/or that
b. the processing unit (3) comprises a heating element and/or a cooling element (36), and/or that
c. the processing unit (3) comprises a further fluid channel (34) in which a heating medium or a coolant can flow, and/or that
d. the container (2) of the carrier (1) is arranged in a receptacle of a receiving element (37), and/or that
e. the processing unit (3) comprises at least one analysis unit (27) for analysing the liquid sample, and/or that
f. the analysis unit (27) comprises at least one sensor and a waste chamber (31) for receiving the portion of the liquid sample sucked into the analysis unit (27), wherein the waste chamber is fluidically connected downstream of the sensor, and/or that
g. the processing unit (3) comprises at least one optical detection apparatus for detecting a property of the liquid sample and/or of the sensor, and/or that
h. the processing unit (3) is configured as a disposable component.

8. The device according to any one of claims 1 to 7, **characterised by** a further processing unit (11) for carrying out at least one further processing step and a further actuating apparatus, which are arranged on the side of the carrier (1) facing away from the processing unit (3).

9. The device according to claim 8, **characterised in that**
a. the further processing unit (11) is arranged between the carrier (1) and the further actuating apparatus, and/or that
b. the further processing unit (11) comprises at least one further optical detection apparatus for detecting a property of the liquid sample and/or of the sensor, and/or that
c. the carrier (1) is mounted on the further processing unit (11), and/or that
d. the further processing unit (11) and/or the actuating apparatus (4) is arranged in a further cavity of a further housing, wherein the further housing is releasably connectable to the carrier (1), and/or that
e. the further actuating apparatus and the actuating apparatus (4) communicate by means of data technology.

10. A method for processing a liquid sample, **characterised in that**
a. at least one container (2) of a carrier (1) is filled with at least one liquid sample, and **in that**
b. a processing unit (3) is mounted on the carrier (1), and that
c. an actuating apparatus (4) is mounted on the processing unit (3), wherein the actuating apparatus (4) comprises an electrical open-loop or closed-loop control unit (5), and that
d. a processing step carried out by the processing unit (3) is controlled in an open-loop or closed-loop manner by the electrical open-loop or closed-loop control unit (5).

11. The method according to claim 10, **characterised in that**
a. a housing which closes off the carrier (1) and the actuating apparatus (4) and the processing unit (3) is releasably connected to the carrier (1), and/or that
b. prior to the processing of the liquid sample, the electrical open-loop or closed-loop control unit (5) is programmed and/or a processing programme is selected and started, and/or that
c. the control of the processing step is carried out according to a predetermined control sequence, and/or that
d. once the processing of the liquid sample has ended, the processing unit (3) is exchanged for another processing unit (11) for carrying out another processing step, in particular and the other processing unit (11) is connected to the same actuating apparatus (4) as the processing unit, or once the processing of the liquid sample has ended, the housing (6) and the actuating apparatus (4) and the processing unit (3) are separated from the carrier (1) and the carrier (1) is transported to a processing station and the liquid sample is processed in the processing station, and/or that
e. the actuating apparatus (4) is transported to a filling station at which a processing medium is fed into a tank of the actuating apparatus (4), and/or that
f. the processing unit (3) is transported to a washing station (49) at which the processing unit (3) is washed, and/or that
g. an assembly or disassembly of the device is carried out manually or automatically, and/or that
h. an assembly or disassembly of the device is carried out in each case by a single handling device, and/or that
i. during assembly of the device a processing unit (3) is selected from a plurality of differently designed processing units and transported to an assembly station in which the processing unit is mounted on the carrier and then an actuating apparatus (4) belonging to the processing unit (3) is selected from a plurality of differently designed actuating apparatuses and transported to the assembly station, in which it is mounted on the processing unit (3), in particular and that once the device has been assembled in the assembly station, the assembled device is transported to a storage station (47), and/or that the transportation of the processing unit (3) and of the actuating apparatus (4) to the individual stations is carried out by the same transporting device (38).

12. The method according to claim 10 or 11, **characterised in that**
a. the liquid sample located in a container (2) of the carrier (1) is optionally mixed by means of the processing unit (3), or a portion of the liquid sample is sucked in or a fluid is dispensed into the liquid sample, in particular and that the mixing of the liquid sample is carried out, that alternately and/or several times in succession a portion of the liquid sample located in the container (2) is sucked into a fluid line of the processing unit (3) and the portion of the liquid sample sucked into the fluid line (19) is then directly dispensed into the liquid sample, and/or that a gas bubble is produced at an outlet of the fluid line (19) and a gas bubble diameter is increased and decreased alternately and/or several times in succession, and/or that
b. at least one mixture is produced by means of the processing unit (3), which mixture comprises at least two processing media, wherein one processing medium is fed in a predetermined amount into the container (2) by means of the processing unit (3) and then another processing medium is fed in a predetermined amount into the same container (2) by means of the processing unit (3), in particular and that the processing medium and the other processing medium are then fed into another container by means of the processing unit (3), wherein the other mixture produced in the other container has a different amount of the processing medium and/or has a different amount of the other processing medium than the mixture located in the container, and/or that
c. a liquid sample is transferred by means of the processing unit (3) from one container (2) into another container, in particular at a constant flow rate, in particular and that the transfer of the liquid sample is carried out in a circulating manner through a plurality of containers (2) or that the transfer of the liquid sample is ended once the liquid sample has been dispensed into a predetermined container (2).

13. The method according to any one of claims 10 to 12, **characterised in that**
a. the container (2) is monitored, in particular by the further optical detection apparatus of a further processing unit (11), wherein a property of the liquid sample is detected optically during the monitoring and the electrical open-loop or closed-loop control unit (5) controls the processing step carried out by the processing unit (3) in a closed-loop manner according to the detected property, in particular and that the mixing ratio in a container (2) is monitored and/or that it is monitored as to whether the liquid sample is contaminated with a foreign body, preferably and that if the liquid sample is contaminated with a foreign body a processing medium, in particular a counteragent, is introduced into the contaminated liquid sample by means of the processing unit (3), and/or that
b. the liquid sample is heated or cooled by means of the processing unit (3), in particular and **in that** a heating medium or a coolant (36) flows through the processing unit (3).

14. The method according to any one of claims 10 to 13, **characterised in that** the processing unit (3) analyses the liquid sample, in particular and that for analysing the liquid sample it is guided to at least one sensor arranged in the processing unit (3), preferably and that the sensor is read out by an optical detection apparatus and/or a further optical detection apparatus, and/or that after the analysis the liquid sample sucked into the processing unit (3) is guided into a waste chamber arranged in the processing unit (3).

## Revendications

1. Dispositif de traitement d'un échantillon liquide, comprenant un support (1) qui comporte au moins un récipient (2) pour recevoir l'échantillon liquide, une unité de traitement (3) pour effectuer au moins une étape de traitement et un dispositif d'actionnement (4) qui a une unité de réglage ou de commande électrique (5) pour commander ou régler l'étape de traitement effectuée au moyen de l'unité de traitement (3), **caractérisé en ce que** le dispositif d'actionnement (4), en particulier l'unité de commande ou de réglage (5), est placée sur l'unité de traitement (3) et **en ce que** l'unité de traitement (3) est placée sur le support (1).

2. Dispositif selon la revendication 1, **caractérisé en ce que** le dispositif présente un boîtier (6)
a. qui est relié de manière amovible au support (1) et ferme le support (1) et/ou en ce que
b. le dispositif d'actionnement (4) est disposé entre le boîtier (6) et le support (1) et/ou **en ce que**
c. le boîtier (6) est placé sur le dispositif d'actionnement (4) et/ou **en ce que**
d. le dispositif est conçu de telle manière que le traitement de l'échantillon liquide se déroule de manière autonome dans le dispositif et/ou **en ce que**
e. le boîtier (6) et/ou le dispositif d'actionnement (4) et/ou l'unité de traitement (3) sont conçus de manière à pouvoir être manipulés par le même dispositif de manipulation que le support (1) et/ou **en ce que**
f. le dispositif d'actionnement (4) est relié de manière amovible à l'unité de traitement (3) et/ou **en ce que**
g. l'unité de traitement (3) est reliée de manière amovible au support (1) et/ou **en ce que**
h. le boîtier (6) est relié de manière amovible ou stationnaire au dispositif d'actionnement (4).

3. Dispositif selon la revendication 1 ou 2, **caractérisé en ce que**
a. le boîtier (6) présente un dispositif de saisie, en particulier un écran tactile (10), pour saisir des données dans l'unité de réglage ou de commande électrique (5) du dispositif d'actionnement (4) et/ou **en ce que**
b. le boîtier (6) est conçu en forme de caisson, le dispositif d'actionnement (4), en particulier l'unité de réglage ou de commande électrique (5), et/ou l'unité de traitement (3) et/ou le support (1) sont disposés dans la cavité du boîtier (6) et/ou **en ce que**
c. le dispositif d'actionnement (4) comporte un dispositif de stockage électrique (14), notamment rechargeable, pour alimenter l'unité de réglage ou de commande électrique (5) en énergie électrique et/ou **en ce que**
d. le dispositif d'actionnement (4) comporte un moyen de communication, notamment sans fil, pour envoyer et/ou recevoir des données et/ou **en ce que**
e. le dispositif d'actionnement (4) comporte au moins une pompe qui peut être commandée par l'unité de réglage ou de commande électrique (5) et/ou **en ce que**
f. le dispositif d'actionnement (4) comporte au moins un réservoir pour stocker un fluide de traitement qui peut être relié de manière fluidique, notamment au moyen d'une vanne, avec une pompe et/ou l'unité de traitement (3) et/ou **en ce que**
g. le dispositif d'actionnement (4) comporte une unité de mesure, en particulier un capteur de pression (18), qui est électriquement connecté à l'unité de réglage ou de commande électrique (5) et/ou est relié de manière fluidique avec l'unité de traitement (3) et/ou **en ce que**
h. le dispositif d'actionnement (4) comporte une plaque (25) sur laquelle sont disposés l'unité de réglage ou de commande électrique (5) et/ou le dispositif de stockage électrique (14) et/ou le moyen de communication et/ou la pompe et/ou le réservoir et/ou l'unité de mesure.

4. Dispositif selon l'une quelconque des revendications 1 à 3, **caractérisé en ce que**
a. lorsque le dispositif d'actionnement (4) est placé sur l'unité de traitement (3) ou lorsque le dispositif d'actionnement (4) est retiré de l'unité de traitement (3), le milieu de traitement se trouvant dans l'unité de traitement (3) et/ou la partie de l'échantillon liquide se trouvant dans l'unité de traitement (3) n'entre pas en contact avec le dispositif d'actionnement (4) et/ou **en ce que**
b. le dispositif d'actionnement (4) est relié à l'unité de traitement (3) sans tube ou sans fil et/ou **en ce que**
c. un élément intermédiaire (7) existant est disposé entre le dispositif d'actionnement (4) et l'unité de traitement (3) et/ou **en ce qu'**
d. un élément intermédiaire (7) existant permet de relier l'unité de traitement (3) au dispositif d'actionnement (4) et/ou **en ce qu'**
e. un élément intermédiaire (7) existant disposé entre le dispositif d'actionnement (4) et l'unité de traitement (3) et/ou permet de relier le dispositif d'actionnement (4) à l'unité de traitement (3), le dispositif d'actionnement (4) étant relié par adhérence de matière à l'unité de traitement (3) au moyen de l'élément intermédiaire (3).

5. Dispositif selon l'une quelconque des revendications 1 à 4, **caractérisé en ce que**
a. l'unité de traitement (3) comporte au moins un autre réservoir pour stocker un autre fluide de traitement et/ou **en ce que**
b. l'unité de traitement (3) comporte au moins une conduite de fluide (19) qui s'étend depuis une pièce rapportée (21) et plonge dans l'échantillon de liquide et/ou **en ce que**
c. l'unité de traitement (3) a au moins une conduite de fluide (19) qui s'étend depuis une pièce rapportée (21) et plonge dans l'échantillon de liquide, au moins une conduite de fluide (19) étant en liaison fluidique avec un canal de fluide (22) disposé dans la pièce rapportée (21) et/ou **en ce que**
d. l'unité de traitement (3) a au moins une conduite de fluide (19) qui s'étend depuis une pièce rapportée (21) et plonge dans l'échantillon, au moins une autre conduite de fluide étant en liaison fluidique avec un autre canal de fluide disposé dans l'accessoire (21).

6. Dispositif selon la revendication 5c ou 5d, **caractérisé en ce que**
a. le canal de fluide (22) est en liaison fluidique avec la pompe et/ou **en ce que**
b. le canal de fluide (22) est en liaison fluidique avec le réservoir et/ou avec l'autre réservoir et/ou **en ce que**
c. l'autre canal de fluide est en liaison fluidique avec une autre pompe et/ou **en ce que**
d. l'autre canal de fluide est en liaison fluidique avec le réservoir et/ou avec l'autre réservoir.

7. Dispositif selon l'une quelconque des revendications 1 à 6, **caractérisé en ce que**
a. l'unité de traitement (3) a au moins une autre vanne qui est reliée à l'unité de réglage ou de commande électrique (5) et/ou **en ce que**
b. l'unité de traitement (3) comporte un élément chauffant et/ou un élément de refroidissement (36) et/ou **en ce que**
c. l'unité de traitement (3) comporte un autre canal de fluide (34) dans lequel peut s'écouler un fluide caloporteur ou un fluide de refroidissement et/ou **en ce que**
d. le récipient (2) du support (1) est disposé dans un logement d'un élément logement (37) et/ou **en ce que**
e. l'unité de traitement (3) comporte au moins une unité d'analyse (27) pour analyser l'échantillon liquide et/ou **en ce que**
f. l'unité d'analyse (27) comporte au moins un capteur et une chambre de rejet (31) pour recevoir la partie de l'échantillon liquide aspiré dans l'unité d'analyse (27), la chambre de rejet étant reliée de manière fluidique en aval du capteur et/ou **en ce que**
g. l'unité de traitement (3) comporte au moins un dispositif de détection optique pour détecter une propriété de l'échantillon liquide et/ou du capteur et/ou **en ce que**
h. l'unité de traitement (3) est conçue comme un composant jetable.

8. Dispositif selon l'une quelconque des revendications 1 à 7, **caractérisé par** une autre unité de traitement (11) pour effectuer au moins une autre étape de traitement et un autre dispositif d'actionnement, qui sont disposés sur le côté du support (1) opposé à l'unité de traitement (3).

9. Dispositif selon la revendication 8, **caractérisé en ce que**
a. l'autre unité de traitement (11) est disposée entre le support (1) et l'autre dispositif d'actionnement et/ou **en ce que**
b. l'autre unité de traitement (11) comporte au moins un autre dispositif de détection optique pour détecter une propriété de l'échantillon liquide et/ou du capteur et/ou **en ce que**
c. le support (1) est placé sur l'unité de traitement supplémentaire (11) et/ou **en ce que**
d. l'autre unité de traitement (11) et/ou le dispositif d'actionnement (4) sont disposés dans une autre cavité d'un autre boîtier, l'autre boîtier pouvant être assemblé de manière amovible au support (1) et/ou **en ce que**
e. l'autre dispositif d'actionnement et le dispositif d'actionnement (4) communiquent de manière informatique.

10. Procédé de traitement d'un échantillon liquide, **caractérisé en ce qu'**
a. au moins un récipient (2) d'un support (1) est rempli d'au moins un échantillon liquide et **en ce qu'**
b. une unité de traitement (3) est placée sur le support (1) et qu'
c. un dispositif d'actionnement (4) est placé sur l'unité de traitement (3), le dispositif d'actionnement (4) ayant une unité de réglage ou de commande électrique (5) et **en ce qu'**
d. une étape de traitement exécutée par l'unité de traitement (3) est commandée ou réglée par l'unité de réglage ou de commande électrique (5).

11. Procédé selon la revendication 10, **caractérisé en ce qu'**
a. un boîtier qui ferme le support (1) et le dispositif d'actionnement (4) et l'unité de traitement (3) est relié de manière amovible au support (1) et/ou **en ce qu'**
b. avant le traitement de l'échantillon liquide, l'unité de réglage ou de commande électrique (5) est programmée et/ou un programme de traitement est sélectionné et démarré et/ou **en ce que**
c. la commande de l'étape de traitement est exécutée selon une séquence de commande prédéfinie et/ou **en ce qu'**
d. après la fin du traitement de l'échantillon liquide, l'unité de traitement (3) est échangée contre une autre unité de traitement (11) pour exécuter une autre étape de traitement, en particulier et l'autre unité de traitement (11) est connectée au même dispositif d'actionnement (4) comme unité de traitement, ou après la fin du traitement de l'échantillon liquide, le boîtier (6) et le dispositif d'actionnement (4) et l'unité de traitement (3) sont séparés du support (1) et le support (1) est transporté vers une station de traitement et l'échantillon liquide est traité dans la station de traitement et/ou **en ce que**
e. le dispositif d'actionnement (4) est transporté vers une station de remplissage où un milieu de traitement est introduit dans un réservoir du dispositif d'actionnement (4) et/ou **en ce que**
f. l'unité de traitement (3) est transportée vers une station de lavage (49) où l'unité de traitement (3) est lavée et/ou **en ce qu'**
g. un montage ou un démontage du dispositif est effectué manuellement ou automatiquement et/ou **en ce qu'**
h. un montage ou un démontage du dispositif est effectué dans chaque cas par un seul dispositif de manutention et/ou **en ce que**
i. lors d'un montage du dispositif à partir d'une pluralité d'unités de traitement de conception différente, une unité de traitement (3) est sélectionnée et transportée vers un poste d'assemblage où l'unité de traitement est placée sur le support, puis un dispositif d'actionnement (4) appartenant à l'unité de traitement (3) est sélectionné parmi une pluralité de dispositifs d'actionnement de conception différente et transporté vers le poste d'assemblage où il est placé sur l'unité de traitement (3), notamment et **en ce qu'**après montage du dispositif dans le poste d'assemblage, le dispositif assemblé est transporté vers un poste de stockage (47) et/ou **en ce que** le transport de l'unité de traitement (3) et du dispositif d'actionnement (4) est effectué par le même dispositif de transport (38) vers les postes individuels.

12. Procédé selon la revendication 10 ou 11, **caractérisé en ce qu'**
a. au moyen de l'unité de traitement (3), l'échantillon liquide se trouvant dans un récipient (2) du support (1) est éventuellement mélangé ou une partie de l'échantillon liquide est aspirée ou un fluide est distribué dans l'échantillon liquide, en particulier et **en ce que** le mélange de l'échantillon liquide est effectué **en ce que**, alternativement et/ou plusieurs fois de suite, une partie de l'échantillon liquide se trouvant dans le récipient (2) est aspirée dans une conduite de fluide de l'unité de traitement (3) et la partie de l'échantillon liquide aspirée dans la conduite de fluide (19) est ensuite directement distribuée dans l'échantillon de liquide et/ou **en ce qu'**une bulle de gaz est générée à une sortie de la conduite de fluide (19) et un diamètre de bulle de gaz est augmenté et diminué alternativement et/ou ou plusieurs fois de suite, et/ou **en ce qu'**
b. au moyen de l'unité de traitement (3), au moins un mélange qui a au moins deux supports de traitement, est généré, au moyen de l'unité de traitement (3), une quantité prédéfinie d'un milieu de traitement étant introduite dans le récipient (2) puis au moyen de l'unité de traitement (3), une quantité prédéfinie d'un autre milieu de traitement ajouté étant introduite dans le même récipient (2), en particulier et **en ce que** le milieu de traitement et l'autre milieu de traitement sont ensuite introduits dans un autre récipient au moyen de l'unité de traitement (3), l'autre mélange généré dans l'autre récipient ayant une quantité différente du milieu de traitement et/ou a une quantité différente de l'autre milieu de traitement que le mélange se trouvant dans le récipient, et/ou en ce qu'
c. au moyen de l'unité de traitement (3) un échantillon liquide est transféré d'un récipient (2) à un autre récipient, en particulier à débit constant, en particulier et **en ce que** le transfert de l'échantillon liquide est effectué de manière circulante à travers plusieurs récipients (2) ou **en ce que** le transfert de l'échantillon liquide se termine après la distribution de l'échantillon liquide dans un récipient prédéfini (2).

13. Procédé selon l'une quelconque des revendications 10 à 12, **caractérisée en ce que**
a. le récipient (2) est surveillé, en particulier par l'autre dispositif de détection optique d'une autre unité de traitement (11), une propriété de l'échantillon liquide étant détectée de manière optique pendant la surveillance et l'unité de réglage ou de commande électrique (5) réglant l'étape de traitement exécutée par l'unité de traitement (3) en fonction de la propriété détectée, en particulier et **en ce que** le rapport de mélange dans un récipient (2) est surveillé et/ou **en ce qu'**il est surveillé si l'échantillon liquide est infecté par un corps étranger, de préférence et **en ce que** si l'échantillon liquide est infecté par un corps étranger, au moyen de l'unité de traitement (3), un milieu de traitement, en particulier un antidote est introduit dans l'échantillon liquide infecté, et/ou **en ce que**
b. l'échantillon liquide est chauffé ou refroidi au moyen de l'unité de traitement (3), en particulier et **en ce qu'**un fluide caloporteur ou un fluide de refroidissement (36) circule à travers l'unité de traitement (3).

14. Procédé selon l'une quelconque des revendications 10 à 13, **caractérisé en ce que** l'unité de traitement(3) analyse notamment l'échantillon de liquide et **en ce que** pour analyser l'échantillon de liquide, il est dirigé vers au moins un capteur disposé dans l'unité de traitement (3), de préférence et **en ce que** le capteur est lu par un dispositif de détection optique et/ou un autre dispositif de détection optique et/ou **en ce que** l'échantillon de liquide aspiré dans l'unité de traitement (3) est dirigé dans une chambre de rejet disposée dans l'unité de traitement (3) après l'analyse.
